(19) Europäisches Patentamt
European Patent Office
Office européen des brevets

(11) EP 3 739 119 A1

(12) **EUROPEAN PATENT APPLICATION**
published in accordance with Art. 153(4) EPC

(43) Date of publication:
**18.11.2020 Bulletin 2020/47**

(21) Application number: **19738261.7**

(22) Date of filing: **08.01.2019**

(51) Int Cl.:
***D21H 27/00*** *(2006.01)* ***A61K 8/02*** *(2006.01)*
***A61K 8/73*** *(2006.01)* ***A61Q 19/00*** *(2006.01)*
***D21H 11/18*** *(2006.01)* ***C08J 9/40*** *(2006.01)*

(86) International application number:
**PCT/JP2019/000214**

(87) International publication number:
**WO 2019/139001 (18.07.2019 Gazette 2019/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.01.2018 JP 2018002081**

(71) Applicant: **Oji Holdings Corporation**
**Chuo-ku**
**Tokyo 104-0061 (JP)**

(72) Inventors:
- **AIZAWA Emi**
  **Tokyo 104-0061 (JP)**
- **SAKAI Koh**
  **Tokyo 104-0061 (JP)**

(74) Representative: **Godemeyer Blum Lenze Patentanwälte**
**Partnerschaft mbB - werkpatent**
**An den Gärten 7**
**51491 Overath (DE)**

(54) **SHEET**

(57) The present invention is intended to provide an ultrafine cellulose fiber-containing wet sheet, which is easily handled upon the use thereof and is excellent in terms of adhesiveness to the skin. The present invention relates to a sheet comprising cellulose fibers having a fiber width of 1000 nm or less and water, wherein the sheet is gelatinous, the cellulose fibers have ionic substituents, the water content percentage is 70% by mass or more with respect to the total mass of the sheet, and the tensile strength is 0.08 MPa or more.

EP 3 739 119 A1

## Description

### Technical Field

The present invention relates to a sheet. Specifically, the present invention relates to a wet sheet comprising ultrafine cellulose fibers.

### Background Art

Conventionally, a cosmetic sheet formed by impregnating a non-woven fabric containing cellulose fibers with a cosmetic component and the like has been known. Such a cosmetic sheet is attached, for example, along the unevenness of the face of a human and is used to enhance cosmetic effects on the skin. In general, such a cosmetic sheet is commercially available in the form of a sheet consisting of a non-woven fabric or the like impregnated with a cosmetic component such as a beauty essence, which is wrapped with a wrapping container.

Upon the use of a cosmetic sheet, it is considered to be preferable that the sheet closely adheres to the skin, and that the permeability of cosmetic components can be felt. For example, Patent Document 1 discloses a cellulose fiber non-woven fabric used for facial masks, in which the cellulose fiber non-woven fabric has a Sin curve pattern and the ratio between wavelength and amplitude and the texture index are within predetermined ranges. This patent document describes that pure water high pressure hydroentanglement is performed in a step of producing the non-woven fabric used for facial masks, so as to adjust the ratio between wavelength and amplitude and the texture index, and that a non-woven fabric used for facial masks, which has high adhesiveness to the skin and is capable of uniformly transferring a liquid medicine into the skin, can be thereby obtained. Moreover, Patent Document 2 discloses a sheet-like cosmetic product containing bio cellulose and a high refractive index water-soluble component. This patent document describes that, by using bio cellulose and a high refractive index water-soluble component, a cosmetic sheet having transparent appearance, regarding which the effect of improving the skin condition can be confirmed by visual observation during the use, can be obtained.

Furthermore, the cosmetic sheet is also required to have properties, by which the impregnated cosmetic components are hardly transpired upon the use thereof. For example, Patent Document 3 discloses a laminated non-woven fabric, in which an extrafine synthetic fiber layer comprising extrafine fibers and a hydrophilic fiber layer are laminated on each other. Further, Patent Document 4 discloses a sheet impregnated with a liquid medicine, in which an ultrafine cellulose fiber non-woven fabric layer is laminated on one surface or both surfaces of a non-woven fabric comprising cellulose fibers. Thus, it has been studied to adjust the fiber type or fiber diameter of each layer constituting a cosmetic sheet, so as to enhance the liquid retention performance of cosmetic components.

### Prior Art Documents

#### Patent Documents

Patent Document 1: Japanese Patent Publication No. 2017-150110 A
Patent Document 2: Japanese Patent Publication No. 2014-111639 A
Patent Document 3: Japanese Patent Publication No. 2005-124916 A
Patent Document 4: Japanese Patent Publication No. 2014-205924 A

### Summary of Invention

#### Object to be Solved by the Invention

It has been desirable for a wet sheet such as, for example, a cosmetic sheet to have properties by which the sheet is not broken and is easily handled. In addition, such a wet sheet has also been required to flexibly follow the unevenness of the face, etc. and to exhibit excellent adhesiveness, depending the intended use thereof. Hence, wet sheets have been required to achieve both handling ability and excellent adhesiveness. However, the wet sheets of the conventional techniques still have had a room for improvement in these performances.

In view of the foregoing, in order to solve the problems of such conventional techniques, the present inventors have conducted studies for the purpose of providing a wet sheet, which is easily handled upon the use thereof and has excellent adhesiveness to the skin.

Means for Solving the Object

As a result of intensive studies directed towards achieving the aforementioned object, the present inventors have found that ultrafine cellulose fibers are used as constitutional materials for a wet sheet and the water content percentage and the tensile strength of a wet sheet are set to be predetermined values or greater, so that a gelatinous wet sheet, which is easily handled upon the use thereof and has excellent adhesiveness to the skin, can be obtained.
Specifically, the present invention has the following configurations.

[1] A sheet comprising cellulose fibers having a fiber width of 1000 nm or less and water, wherein
the sheet is gelatinous,
the cellulose fibers have ionic substituents,
the water content percentage is 70% by mass or more with respect to the total mass of the sheet, and
the tensile strength is 0.08 MPa or more.
[2] The sheet according to [1], wherein the fiber width of the cellulose fibers is 8 nm or less.
[3] The sheet according to [1] or [2], which has a tensile elastic modulus of 0.5 MPa or more.
[4] The sheet according to any one of [1] to [3], which has an elongation of 5.0% or more.
[5] The sheet according to any one of [1] to [4], which has a haze of 20.0% or less.
[6] The sheet according to any one of [1] to [5], wherein the density of the sheet in an absolute dry state is 0.5 $g/cm^3$ or more.
[7] The sheet according to any one of [1] to [6], wherein the ionic substituents are phosphoric acid groups or phosphoric acid group-derived substituents.
[8] The sheet according to any one of [1] to [7], which further comprises a resin component.
[9] The sheet according to any one of [1] to [8], which further comprises an external preparation for skin.
[10] The sheet according to any one of [1] to [9], which is used as a cosmetic sheet.

Effects of Invention

According to the present invention, a gelatinous wet sheet, which is easily handled upon the use thereof and has excellent adhesiveness to the skin, can be obtained.

Brief Description of Drawings

Figure 1 is a graph showing the relationship between the amount of NaOH added dropwise to a fiber raw material having phosphoric acid groups and electrical conductivity.
Figure 2 is a graph showing the relationship between the amount of NaOH added dropwise to a fiber raw material having carboxyl groups and electrical conductivity.

Embodiments of Carrying out the Invention

Hereinafter, the present invention will be described in detail. The description for components described below will be based on representative embodiments or specific examples; however, the present invention will not be limited to such embodiments.

(Ultrafine cellulose fiber-containing wet sheet)

The present invention relates to a gelatinous sheet comprising cellulose fibers having a fiber width of 1000 nm or less and water. Herein, the cellulose fibers have ionic substituents. In addition, the water content percentage of the sheet is 70% by mass or more, with respect to the total mass of the sheet, and the tensile strength of the sheet is 0.08 MPa or more.
Besides, in the present description, the cellulose fibers having a fiber width of 1000 nm or less may also be referred to as "ultrafine cellulose fibers." Moreover, in the present description, a gelatinous sheet may also be referred to as a "wet sheet" or an "ultrafine cellulose fiber-containing wet sheet."
Since the sheet of the present invention has the above-described configuration, it is easily handled upon the use thereof, and it is also excellent in terms of adhesiveness to the skin. Specifically, since the sheet of the present invention is a sheet having moderate elongation and strength, it is easily attached along the unevenness structure of a face, or the like. Furthermore, in the sheet of the present invention, air bubbles jammed between the sheet and a target

adherend can be easily found by visual observation, and also, the jammed air bubbles can be easily removed. Thus, it is easy to closely adhere the sheet to the target adherend. Further, after the sheet of the present invention has been attached to a target adherend, a state in which the sheet is closely adhered to the target adherend, is maintained. Hence, even in a case where some motion occurs in the uneven structure, a part of the sheet is neither floated nor peeled. As such, the sheet of the present invention has both the ease of handling and excellent adhesiveness. Besides, in the present description, the "state in which the present sheet is excellent in terms of adhesiveness" means a state in which the sheet has favorable initial adhesiveness by which the sheet can be closely adhered to a target adherend without air bubbles jammed between the sheet and the target adherend, and also in which after the attachment of the sheet, the sheet can exhibit adhesion persistence, by which the sheet can maintain a closely adhered state.

In order to obtain a wet sheet having the above-described configuration, it is preferable to appropriately select, for example, the composition of the sheet or the production process. For example, one aspect of the process capable of realizing the above-described configuration is that a wet sheet is obtained by adding water to an ultrafine cellulose fiber-containing sheet that has been once dried. This is considered because an ultrafine cellulose fiber-containing sheet is once dried and is then converted to a wet state, so that water can be uniformly distributed in the sheet, thereby satisfying the above-described configuration.

In one embodiment of the present invention, it is also preferable to obtain a wet sheet comprising crosslinked ultrafine cellulose fibers with the use of metal salts. In this case, in order to obtain a wet sheet having the above-described configuration, the time required for the crosslinking reaction of ultrafine cellulose fibers with metal salts is adjusted, so that the degree of crosslinking is favorably set within a suitable range. For example, by reducing the time for immersing ultrafine cellulose fibers in a solution containing metal salts, a wet sheet having a low degree of crosslinking is favorably obtained. Besides, in a wet sheet comprising crosslinked ultrafine cellulose fibers with the use of metal salts, the water absorption rate can be suppressed. Accordingly, when water is added to a dried ultrafine cellulose fiber-containing sheet, the time for immersing the sheet in water can be set to be relatively long. Thereby, when the dried ultrafine cellulose fiber-containing sheet is immersed in water, it also becomes possible to provide ranges to the immersion time, so that process control can be easily carried out.

The sheet of the present invention is further excellent in terms of transparency. Specifically, the haze of the sheet of the present invention is preferably 20.0% or less, more preferably 15.0% or less, even more preferably 10.0% or less, further preferably 7.5% or less, still further preferably 6.7% or less, particularly preferably 6.5% or less, and most preferably 6.0% or less. It is to be noted that the haze of a sheet is a value measured in accordance with JIS K 7136, for example, using a hazemeter (manufactured by MURAKAMI COLOR RESEARCH LABORATORY Co., Ltd.; HM-150). Thus, since the sheet of the present invention is also excellent in terms of transparency, even in a case where air bubbles are penetrated between a target adherend and the sheet upon the attachment of the sheet, such penetration of the air bubbles can be confirmed from the sheet side by visual observation. Thereby, a sheet user may perform an operation to closely adhere the sheet to the target adherend such that the air bubbles are removed, or may attach the sheet again, and as a result, the adhesiveness of the sheet to the target adherend can be more effectively enhanced.

The total light transmittance of the sheet of the present invention is preferably 70% or more, more preferably 80% or more, and further preferably 85% or more. It is to be noted that the total light transmittance of a sheet is a value measured in accordance with JIS K 7361, using, for example, a hazemeter (manufactured by MURAKAMI COLOR RESEARCH LABORATORY Co., Ltd.; HM-150).

The sheet of the present invention is a gelatinous sheet. In the present description, whether the sheet is a gelatinous sheet can be judged based on the fact that even in a case where a wet sheet having a water content percentage of 70% by mass or more is pressurized, a liquid is not dripped from the sheet. The rubber hardness of the sheet is preferably E1/30 or more, and more preferably E5/30 or more. On the other hand, the rubber hardness of the sheet is preferably E90/30 or less. It is to be noted that, in general, such rubber hardness cannot be measured for non-woven fabric sheets. Thus, when the rubber hardness of the wet sheet is within the above-described range, the wet sheet of the present invention can be distinguished from non-woven fabric sheets.

The water content percentage of the sheet of the present invention may be 70% by mass or more, with respect to the total mass of the sheet, and it is preferably 75% by mass or more, more preferably 80% by mass or more, and further preferably 85% by mass or more. On the other hand, the water content percentage of the sheet is preferably 95% by mass or less, with respect to the total mass of the sheet. By setting the water content percentage of the sheet within the above-described range, the adhesiveness of the wet sheet to the skin can be enhanced.

The solid content in the sheet of the present invention is preferably 5% by mass or more, and more preferably 10% by mass or more, with respect to the total mass of the sheet. On the other hand, the solid content is preferably 30% by mass or less, more preferably 25% by mass or less, further preferably 20% by mass or less, and particularly preferably 15% by mass or less, with respect to the total mass of the sheet. By setting the solid content in the sheet within the above-described range, the adhesiveness of the wet sheet to the skin can be enhanced.

The water content percentage of the sheet of the present invention is preferably 233% by mass or more, more preferably 300% by mass or more, further preferably 400% by mass or more, and particularly preferably 565% by mass

or more, with respect to 100% by mass of the solid content mass. On the other hand, the water content percentage is preferably 1920% by mass or less, with respect to 100% by mass of the solid content mass. By setting the water content percentage of the sheet within the above-described range, a wet sheet having excellent adhesiveness to the skin can be obtained.

The tensile strength of the sheet of the present invention (wet sheet) may be 0.08 MPa or more, and it is preferably 0.1 MPa or more, more preferably 0.2 MPa or more, further preferably 0.4 MPa or more, and particularly preferably 0.5 MPa or more. On the other hand, the tensile strength of the sheet (wet sheet) is preferably 10.0 MPa or less, more preferably 8.0 MPa or less, and further preferably 6.0 MPa or less. By setting the tensile strength of the sheet (wet sheet) within the above-described range, it becomes easy to attach the sheet to a target adherend such as a face upon the use thereof, and thus, handling ability becomes favorable.

The tensile elastic modulus of the sheet of the present invention (wet sheet) is preferably 0.5 MPa or more, more preferably 0.8 MPa or more, and further preferably 1.0 MPa or more. On the other hand, the tensile elastic modulus of the sheet (wet sheet) is preferably 50.0 MPa or less, more preferably 40.0 MPa or less, and further preferably 30.0 MPa or less. By setting the tensile elastic modulus of the sheet (wet sheet) within the above-described range, it becomes easy to attach the sheet to a target adherend such as a face upon the use thereof, and thus, handling ability becomes favorable. Moreover, by setting the tensile elastic modulus of the sheet (wet sheet) within the above-described range, the adhesiveness of the sheet to a target adherend can be more effectively enhanced.

The elongation of the sheet of the present invention (wet sheet) is preferably 5.0% or more, more preferably 6.0% or more, further preferably 7.0% or more, and particularly preferably 10.0% or more. On the other hand, the elongation of the sheet (wet sheet) is preferably 50.0% or less, more preferably 40.0% or less, and further preferably 30.0% or less. By setting the elongation of the sheet (wet sheet) within the above-described range, it becomes easy to attach the sheet to a target adherend such as a face upon the use thereof, and thus, handling ability becomes favorable. Moreover, by setting the elongation of the sheet (wet sheet) within the above-described range, the adhesiveness of the sheet to a target adherend can be more effectively enhanced.

Herein, the tensile strength, tensile elastic modulus and elongation of the sheet mean the tensile strength, tensile elastic modulus and elongation of a gelatinous wet sheet. Specifically, the tensile strength, tensile elastic modulus and elongation of the sheet are values obtained by cutting a wet sheet into a size of 25 mm wide and 150 mm long, then setting a distance between holders at 100 mm, and then measuring the tensile strength, tensile elastic modulus and elongation in accordance with JIS P 8135. For the measurement, for example, a tension testing machine "Tensilon" (manufactured by A & D Company, Limited) can be used.

The content of cellulose fibers is preferably 1% by mass or more, more preferably 10% by mass or more, and further preferably 20% by mass or more, with respect to the solid content mass in the sheet of the present invention. On the other hand, the content of cellulose fibers is preferably 90% by mass or less, with respect to the solid content mass in the sheet.

The thickness of the sheet of the present invention (wet sheet) is not particularly limited, and it is preferably 5 μm or more, more preferably 10 μm or more, and further preferably 20 μm or more. On the other hand, the thickness of the wet sheet is preferably 2000 μm or less. The thickness of the wet sheet can be measured, for example, using a constant pressure thickness gauge (manufactured by TECLOCK Co., Ltd.).

The thickness of a sheet that is in an absolute dry state (dry sheet), which is obtained by drying the sheet of the present invention, is not particularly limited, and it is preferably 5 μm or more, more preferably 10 μm or more, and further preferably 20 μm or more. On the other hand, the thickness of the dry sheet is preferably 1000 μm or less. The thickness of the dry sheet can be measured, for example, using a constant pressure thickness gauge (manufactured by TECLOCK Co., Ltd.).

The basis weight of the sheet that is in an absolute dry state (dry sheet) is preferably 5 $g/m^2$ or more, more preferably 10 $g/m^2$ or more, and further preferably 20 $g/m^2$ or more. On the other hand, the basis weight of the dry sheet is preferably 300 $g/m^2$ or less. Herein, the basis weight of the sheet can be calculated, for example, in accordance with JIS P 8124.

The density of the sheet that is in an absolute dry state (dry sheet) is preferably 0.5 $g/cm^3$ or more, more preferably 0.7 $g/cm^3$ or more, and further preferably 1.0 $g/cm^3$ or more. On the other hand, the density of the dry sheet is preferably 2.0 $g/cm^3$ or less. It is to be noted that the density of the dry sheet is a value calculated from the aforementioned thickness and basis weight of the dry sheet.

(Ultrafine cellulose fibers)

The sheet of the present invention comprises cellulose fibers having a fiber width of 1000 nm or less and having ionic substituents. The fiber width of cellulose fibers is preferably 100 nm or less, more preferably 50 nm or less, further preferably 10 nm or less, and particularly preferably 8 nm or less. The fiber width of cellulose fibers can be measured, for example, by electron microscopic observation.

The average fiber width of the cellulose fibers is, for example, 1000 nm or less. The average fiber width of the cellulose fibers is, for example, preferably 2 nm or more and 1000 nm or less, more preferably 2 nm or more and 100 nm or less, further preferably 2 nm or more and 50 nm or less, still further preferably 2 nm or more and 10 nm or less, and particularly preferably 2 nm or more and 8 nm or less. By setting the average fiber width of the cellulose fibers at 2 nm or more, dissolution of the cellulose fibers as cellulose molecules in water is suppressed, and the effects of the cellulose fibers, such as the improvement of strength, rigidity, and dimensional stability, can be easily expressed. It is to be noted that the cellulose fibers are, for example, monofibrous cellulose.

The average fiber width of cellulose fibers is measured as follows, for example, using an electron microscope. First, an aqueous suspension of cellulose fibers having a concentration of 0.05% by mass or more and 0.1% by mass or less is prepared, and this suspension is casted onto a hydrophilized carbon film-coated grid as a sample for TEM observation. If the sample contains wide fibers, SEM images of the surface of the suspension casted onto glass may be observed. Subsequently, the sample is observed using electron microscope images taken at a magnification of 1000x, 5000x, 10000x, or 50000x, depending on the widths of fibers used as observation targets. However, the sample, the observation conditions, and the magnification are adjusted so as to satisfy the following conditions:

(1) A single straight line X is drawn in any given portion in an observation image, and 20 or more fibers intersect with the straight line X.
(2) A straight line Y, which intersects perpendicularly with the aforementioned straight line in the same image as described above, is drawn, and 20 or more fibers intersect with the straight line Y.

The widths of the fibers intersecting the straight line X and the straight line Y in the observation image meeting the above-described conditions are visually read. Three or more sets of observation images of surface portions, which are at least not overlapped, are obtained. Thereafter, the widths of the fibers intersecting the straight line X and the straight line Y are read in each image. Thereby, at least 120 fiber widths (20 fibers $\times$ 2 $\times$ 3 = 120) are thus read. The average value of the read fiber widths is defined to be the average fiber width of cellulose fibers.

The fiber length of the cellulose fibers is not particularly limited, and for example, it is preferably 0.1 $\mu$m or more and 1000 $\mu$m or less, more preferably 0.1 $\mu$m or more and 800 $\mu$m or less, and further preferably 0.1 $\mu$m or more and 600 $\mu$m or less. By setting the fiber length within the above-described range, destruction of the crystalline region of the cellulose fibers can be suppressed. In addition, the viscosity of a slurry of the cellulose fibers can also be set within an appropriate range. It is to be noted that the fiber length of the cellulose fibers can be obtained by an image analysis using TEM, SEM or AFM.

The cellulose fibers preferably have a type I crystal structure. Herein, the fact that the cellulose fibers have a type I crystal structure may be identified by a diffraction profile obtained from a wide angle X-ray diffraction photograph using CuK$\alpha$ ($\lambda$ = 1.5418 Å) monochromatized with graphite. Specifically, it may be identified based on the fact that there are typical peaks at two positions near $2\theta$ = 14° or more and 17° or less, and near $2\theta$ = 22° or more and 23° or less.

The percentage of the type I crystal structure occupied in the ultrafine cellulose fibers is, for example, preferably 30% or more, more preferably 40% or more, and further preferably 50% or more. Thereby, more excellent performance can be expected, in terms of heat resistance and the expression of low linear thermal expansion. The crystallinity can be obtained by measuring an X-ray diffraction profile and obtaining it according to a common method (Seagal et al., Textile Research Journal, Vol. 29, p. 786, 1959).

The aspect ratio (fiber length/fiber width) of the cellulose fibers is not particularly limited, and for example, it is preferably 20 or more and 10000 or less, and more preferably 50 or more and 1000 or less. By setting the aspect ratio at the above-described lower limit value or more, a sheet comprising ultrafine cellulose fibers is easily formed. Moreover, sufficient thickening properties are easily obtained upon production of a dispersed form in a solvent. By setting the aspect ratio at the above-described upper limit or less, when the cellulose fibers are treated, for example, as an aqueous dispersed solution, operations such as dilution are preferably easily handled.

The cellulose fibers in the present embodiment have, for example, both a crystalline region and an amorphous region. In particular, ultrafine cellulose fibers, which have both a crystalline region and an amorphous region and also have a high aspect ratio, are realized by the after-mentioned method for producing ultrafine cellulose fibers.

The cellulose fibers have ionic substituents. The cellulose fibers may comprise, as such ionic substituents, for example, either anionic functional groups or cationic functional groups, or both of them. In the present embodiment, the cellulose fibers preferably have anionic functional groups as ionic substituents.

The anionic functional groups are preferably at least one type selected from, for example, phosphoric acid groups or phosphoric acid group-derived substituents (which are simply referred to as "phosphoric acid groups" at times), carboxyl groups or carboxyl group-derived substituents (which are simply referred to as "carboxyl groups" at times), and sulfone groups or sulfone group-derived substituents (which are simply referred to as "sulfone groups" at times); are more preferably at least one type selected from phosphoric acid groups and carboxyl groups; and are particularly preferably phosphoric acid groups. When the cellulose fibers have phosphoric acid groups, a highly transparent sheet

can be easily obtained.

The phosphoric acid group is a divalent functional group corresponding to, for example, a phosphoric acid from which a hydroxyl group is removed. Specifically, it is a group represented by $-PO_3H_2$. The phosphoric acid group-derived substituents include substituents, such as salts of phosphoric acid groups and phosphoric acid ester groups. Besides, the phosphoric acid group-derived substituents may be comprised as condensed phosphoric acid groups (for example, pyrophosphoric acid groups) in the cellulose fibers.

The phosphoric acid group or the phosphoric acid group-derived substituent may be a substituent represented by, for example, the following Formula (1):

[Formula 1]

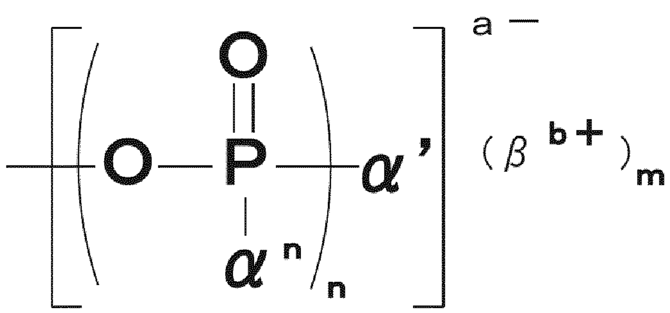

(1)

In the above Formula (1), a, b, and n each represent a natural number (provided that a = b x m); an "a" number of $\alpha^1$, $\alpha^2$, ..., $\alpha^n$ and $\alpha'$ is $O^-$, and the rest is either R or OR. All of $\alpha^n$ and $\alpha'$ may also be $O^-$. R each represents a hydrogen atom, a saturated straight chain hydrocarbon group, a saturated branched chain hydrocarbon group, a saturated cyclic hydrocarbon group, an unsaturated straight chain hydrocarbon group, an unsaturated branched chain hydrocarbon group, an unsaturated cyclic hydrocarbon group, an aromatic group, or a derivative group thereof.

Examples of the saturated straight chain hydrocarbon group may include a methyl group, an ethyl group, an n-propyl group, and an n-butyl group, but are not particularly limited thereto. Examples of the saturated branched chain hydrocarbon group may include an i-propyl group and a t-butyl group, but are not particularly limited thereto. Examples of the saturated cyclic hydrocarbon group may include a cyclopentyl group and a cyclohexyl group, but are not particularly limited thereto. Examples of the unsaturated straight chain hydrocarbon group may include a vinyl group and an allyl group, but are not particularly limited thereto. Examples of the unsaturated branched chain hydrocarbon group may include an i-propenyl group and a 3-butenyl group, but are not particularly limited thereto. Examples of the unsaturated cyclic hydrocarbon group may include a cyclopentenyl group and a cyclohexenyl group, but are not particularly limited thereto. Examples of the aromatic group may include a phenyl group and a naphthyl group, but are not particularly limited thereto.

Moreover, examples of the derivative group of the R may include functional groups such as a carboxyl group, a hydroxyl group or an amino group, in which at least one type selected from the functional groups is added to or substituted with the main chain or side chain of the above-described various types of hydrocarbon groups, but are not particularly limited thereto. Furthermore, the number of carbon atoms constituting the main chain of the above-described R is not particularly limited, and it is preferably 20 or less, and more preferably 10 or less. By setting the number of carbon atoms constituting the main chain of the R within the above-described range, the molecular weight of phosphoric acid groups can be adjusted in a suitable range, permeation thereof into a fiber raw material can be facilitated, and the yield of the ultrafine cellulose fibers can also be enhanced.

$\beta^{b+}$ is a mono- or more-valent cation consisting of an organic or inorganic matter. Examples of the mono- or more-valent cation consisting of an organic matter may include an aliphatic ammonium and an aromatic ammonium, and examples of the mono- or more-valent cation consisting of an inorganic matter may include alkali metal ions such as sodium, potassium or lithium ions, divalent metal cations such as calcium or magnesium ions, and hydrogen ions, but are not particularly limited thereto. These can be applied alone as a single type or in combination of two or more types. As such mono- or more-valent cations consisting of an organic or inorganic matter, sodium or potassium ions, which hardly cause the yellowing of a fiber raw material containing $\beta$ upon heating and are industrially easily applicable, are preferable, but are not particularly limited thereto.

The amount of anionic functional groups introduced into the cellulose fibers is, for example, per 1 g (mass) of the cellulose fibers, preferably 0.10 mmol/g or more, more preferably 0.20 mmol/g or more, further preferably 0.50 mmol/g or more, and particularly preferably 1.00 mmol/g or more. On the other hand, the amount of anionic functional groups introduced into the cellulose fibers is, for example, per 1 g (mass) of the ultrafine cellulose fibers, preferably 3.65 mmol/g

or less, more preferably 3.50 mmol/g or less, and further preferably 3.00 mmol/g or less. By setting the amount of anionic functional groups introduced within the above-described range, it can become easy to perform fibrillation on the fiber raw material, and the stability of the cellulose fibers can be enhanced. In addition, by setting the amount of anionic functional groups introduced within the above-described range, favorable properties can be exhibited in a sheet comprising the cellulose fibers, etc.

Herein, the denominator in the unit mmol/g indicates the mass of cellulose fibers, when the counterions of anionic functional groups are hydrogen ions ($H^+$).

The amount of anionic functional groups introduced into the cellulose fibers may be measured, for example, by a conductometric titration method. In the measurement according to the conductometric titration method, while an alkali such as a sodium hydroxide aqueous solution is added to a slurry containing the obtained cellulose fibers, a change in the electrical conductivity is obtained, so that the amount of anionic functional groups introduced is measured.

Figure 1 is a graph showing the relationship between the amount of NaOH added dropwise to cellulose fibers having phosphoric acid groups and electrical conductivity. The amount of the phosphoric acid groups introduced into the cellulose fibers is measured, for example, as follows. First, a slurry containing cellulose fibers is treated with a strongly acidic ion exchange resin. Before the treatment with the strongly acidic ion exchange resin, the same defibration treatment as the after-mentioned defibration treatment may be performed on the cellulose fibers, as necessary. Subsequently, while adding a sodium hydroxide aqueous solution, a change in the electrical conductivity is observed, and a titration curve as shown in Figure 1 is obtained. As shown in Figure 1, first, the electrical conductivity is rapidly reduced (hereinafter, this region is referred to as a "first region"). Then, the conductivity starts rising slightly (hereinafter, this region is referred to as a "second region"). Then, the increment of the conductivity is further increased (hereinafter, this region is referred to as a "third region"). The boundary point between the second region and the third region is defined as a point at which a change amount in the two differential values of conductivity, namely, an increase in the conductivity (inclination) becomes maximum. Thus, three regions appear in the titration curve. Among them, the amount of the alkali required for the first region among these regions is equal to the amount of a strongly acidic group in the slurry used in the titration, and the amount of the alkali required for the second region is equal to the amount of a weakly acidic group in the slurry used in the titration. When condensation of a phosphoric acid group occurs, the weakly acidic group is apparently lost, so that the amount of the alkali required for the second region is decreased as compared with the amount of the alkali required for the first region. On the other hand, the amount of the strongly acidic group agrees with the amount of the phosphorus atom regardless of the presence or absence of condensation. Hence, the simple term "the amount of the phosphoric acid group introduced (or the amount of the phosphoric acid group)" or "the amount of the substituent introduced (or the amount of the substituent)" refers to the amount of the strongly acidic group. Therefore, the value obtained by dividing the amount (mmol) of the alkali required for the first region in the titration curve as obtained above by the solid content (g) in the slurry as a titration target becomes the amount (mmol/g) of the phosphoric acid groups introduced.

Figure 2 is a graph showing the relationship between the amount of NaOH added dropwise to cellulose fibers having carboxyl groups and electrical conductivity. The amount of the carboxyl groups introduced into the cellulose fibers is measured, for example, as follows. First, a slurry containing cellulose fibers is treated with a strongly acidic ion exchange resin. Before the treatment with the strongly acidic ion exchange resin, the same defibration treatment as the after-mentioned defibration treatment may be performed on the cellulose fibers, as necessary. Subsequently, while adding a sodium hydroxide aqueous solution, a change in the electrical conductivity is observed, and a titration curve as shown in Figure 2 is obtained. As shown in Figure 2, the titration curve is divided into a first region that corresponds to until an increment (inclination) in the electric conductivity becomes almost constant after the electric conductivity has been reduced, and a second region that corresponds to until an increment (inclination) in the conductivity is increased. It is to be noted that the boundary point between the first region and the second region is defined as a point at which the second-order differential value of the conductivity, namely, the amount of change in the increment (inclination) in the conductivity, becomes maximum. The value obtained by dividing the amount (mmol) of the alkali required for the first region in the titration curve by the solid content (g) in the ultrafine cellulose fiber-containing slurry as a titration target is defined to be the amount (mmol/g) of carboxyl groups introduced.

Regarding the aforementioned amount (mmol/g) of carboxyl groups introduced, since the denominator indicates the mass of acid-type cellulose fibers, the amount (mmol/g) of carboxyl groups introduced indicates the amount of carboxyl groups possessed by the acid-type cellulose fibers (hereinafter referred to as "the amount of carboxyl group (acid type)"). On the other hand, when the counterions of carboxyl groups are substituted with any given cations C to achieve charge equivalent, the denominator is converted to the mass of cellulose fibers in which cations C are counterions, so that the amount of carboxyl groups possessed by the cellulose fibers in which the cations C are counterions (hereinafter referred to as "the amount of carboxyl groups (C type)") can be obtained.

Specifically, the amount of carboxyl groups introduced is calculated according to the following equation:

$$\text{Amount of carboxyl groups (C type) introduced} = \text{amount of carboxyl groups (acid type)} / \{1 + (W\text{-}1) \times (\text{amount of carboxyl groups (acid type)}) / 1000\}.$$

In the equation, W indicates formula weight per valence of cations C (for example, Na: 23; and Al: 9).

<Step of producing ultrafine cellulose fibers >

< Fiber raw material >

Ultrafine cellulose fibers are produced from a fiber raw material comprising cellulose. Such a fiber raw material comprising cellulose is not particularly limited, and pulp is preferably used from the viewpoint of availability and inexpensiveness. Examples of the pulp may include wood pulp, non-wood pulp, and deinked pulp. Examples of the wood pulp may include, but are not particularly limited to, chemical pulps such as leaf bleached kraft pulp (LBKP), needle bleached kraft pulp (NBKP), sulfite pulp (SP), dissolving pulp (DP), soda pulp (AP), unbleached kraft pulp (UKP), and oxygen bleached kraft pulp (OKP); semichemical pulps such as semi-chemical pulp (SCP) and chemi-ground wood pulp (CGP); and mechanical pulps such as ground pulp (GP) and thermomechanical pulp (TMP, BCTMP). Examples of the non-wood pulp may include, but not particularly limited to, cotton pulps such as cotton linter and cotton lint; and non-wood type pulps such as hemp, wheat straw, and bagasse. An example of a deinked pulp may be, but is not particularly limited to, a deinked pulp using waste paper as a raw material. The pulp of the present embodiment may be used alone as a single type, or in combination of two or more types.

Among the above-listed pulps, for example, wood pulp and deinked pulp are preferable from the viewpoint of easy availability. Moreover, among wood pulps, for example, chemical pulp is more preferable, and kraft pulp and sulfite pulp are further preferable, from the viewpoint that it has a higher cellulose content ratio so as to enhance the yield of ultrafine cellulose fibers upon the defibration treatment, and that decomposition of cellulose in the pulp is mild, so that ultrafine cellulose fibers having a long fiber length with a high aspect ratio can be obtained.

As a fiber raw material comprising cellulose, for example, cellulose comprised in Ascidiacea, or bacterial cellulose generated by acetic acid bacteria can also be utilized. In addition fibers formed from straight-chain nitrogen-containing polysaccharide polymers such as chitin and chitosan can also be used, instead of a fiber raw material containing cellulose.

< Phosphoric acid group introduction step >

The phosphoric acid group introduction step is a step of reacting at least one compound selected from compounds capable of introducing phosphoric acid groups (hereinafter also referred to as "Compound A") with a hydroxyl group of a fiber raw material comprising cellulose, so that the compound is allowed to act on the fiber raw material comprising cellulose. By this step, phosphoric acid group-introduced fibers can be obtained.

In the phosphoric acid group introduction step according to the present embodiment, the reaction of the fiber raw material comprising cellulose with Compound A may be carried out in the presence of at least one type selected from urea and a derivative thereof (hereinafter also referred to as "Compound B"). Otherwise, the reaction of the fiber raw material comprising cellulose with Compound A may also be carried out in the absence of Compound B.

One example of the method of allowing Compound A to act on the fiber raw material in the presence of Compound B may include a method of mixing Compound A and Compound B into the fiber raw material that is in a dry or wet state, or in a slurry state. Among the fiber raw materials in these states, because of the high uniformity of the reaction, the fiber raw material that is in a dry or wet state is preferably used, and the fiber raw material in a dry state is particularly preferably used. The shape of the fiber raw material is not particularly limited, and for example, a cotton-like or thin sheet-like fiber raw material is preferable. Compound A and Compound B may be added to the fiber raw material by the method of adding Compound A and Compound B that are dissolved in a solvent to form a solution, or are melted by being heated to a melting point or higher. Among these, because of the high uniformity of the reaction, the compounds are preferably added to the fiber raw material, in the form of a solution obtained by dissolution thereof in a solvent, or in particular, in the form of an aqueous solution. Moreover, Compound A and Compound B may be simultaneously added, or may also be added, separately. Alternatively, Compound A and Compound B may be added in the form of a mixture thereof. The method of adding Compound A and Compound B is not particularly limited, and in a case where Compound A and Compound B are in the form of a solution, the fiber raw material may be immersed in the solution for liquid absorption, and may be then removed therefrom, or the solution may also be added dropwise onto the fiber raw material. Otherwise, Compound A and Compound B in necessary amounts may be added to the fiber raw material, or Compound A and Compound B in excessive amounts may be added to the fiber raw material and then, may be squeezed or filtrated to remove redundant Compound A and Compound B.

Examples of Compound A used in the present embodiment may include phosphoric acid or a salt thereof, dehydrated condensed phosphoric acid or a salt thereof, and phosphoric anhydride (diphosphorus pentoxide), but are not particularly limited thereto. As such phosphoric acid, those having various purities can be used, and for example, 100% phosphoric acid (orthophosphoric acid) or 85% phosphoric acid can be used. Dehydrated condensed phosphoric acid is phosphoric acid that is condensed by two or more molecules according to a dehydration reaction, and examples of such dehydrated condensed phosphoric acid may include pyrophosphoric acid and polyphosphoric acid. Examples of the phosphate and salts of dehydrated condensed phosphoric acid may include lithium salts, sodium salts, potassium salts, and ammonium salts of phosphoric acid or dehydrated condensed phosphoric acid, and these salts may have various neutralization degrees. Among these, from the viewpoints of high efficiency in introduction of the phosphoric acid groups, an improving tendency of the defibration efficiency in a defibration step described below, low costs, and industrial applicability, phosphoric acid, sodium salts of phosphoric acid, potassium salts of phosphoric acid, or ammonium salts of phosphoric acid are preferable, and phosphoric acid, sodium dihydrogen phosphate, disodium hydrogen phosphate, or ammonium dihydrogen phosphate is more preferable.

The amount of Compound A added to the fiber raw material is not particularly limited, and for example, if the amount of the Compound A added is converted to a phosphorus atomic weight, the amount of phosphorus atoms added with respect to the fiber raw material (absolute dry mass) is preferably 0.5% by mass or more and 100% by mass or less, more preferably 1% by mass or more and 50% by mass or less, and further preferably 2% by mass or more and 30% by mass or less. By setting the amount of phosphorus atoms added to the fiber raw material within the above-described range, the yield of the ultrafine cellulose fibers can be further improved. On the other hand, by setting the amount of phosphorus atoms added to the fiber raw material to the above-described upper limit value or less, the balance between the effect of improving the yield and costs can be kept.

Compound B used in the present embodiment is at least one type selected from urea and a derivative thereof, as described above. Examples of Compound B may include urea, biuret, 1-phenyl urea, 1-benzyl urea, 1-methyl urea, and 1-ethyl urea. From the viewpoint of the improvement of the uniformity of the reaction, Compound B is preferably used in the form of an aqueous solution. Moreover, from the viewpoint of the further improvement of the uniformity of the reaction, an aqueous solution, in which both Compound A and Compound B are dissolved, is preferably used.

The amount of Compound B added to the fiber raw material (absolute dry mass) is not particularly limited, and for example, it is preferably 1% by mass or more and 500% by mass or less, more preferably 10% by mass or more and 400% by mass or less, and further preferably 100% by mass or more and 350% by mass or less.

In the reaction of the fiber raw material comprising cellulose with Compound A, for example, amides or amines, as well as Compound B, may be comprised in the reaction system. Examples of the amides may include formamide, dimethylformamide, acetamide, and dimethylacetamide. Examples of the amines may include methylamine, ethylamine, trimethylamine, triethylamine, monoethanolamine, diethanolamine, triethanolamine, pyridine, ethylenediamine, and hexamethylenediamine. Among these, particularly, triethylamine is known to work as a favorable reaction catalyst.

In the phosphoric acid group introduction step, after Compound A, etc. is added or mixed into the fiber raw material, a heat treatment is preferable performed on the fiber raw material. For the temperature of such a heat treatment, it is preferable to select a temperature that allows an efficient introduction of phosphoric acid groups, while suppressing the thermal decomposition or hydrolysis reaction of fibers. For example, the heat treatment temperature is preferably 50°C or higher and 300°C or lower, more preferably 100°C or higher and 250°C or lower, and further preferably 130°C or higher and 200°C or lower. In addition, apparatuses having various heating media can be utilized in the heat treatment, and examples of such an apparatus may include a stirring dryer, a rotary dryer, a disk dryer, a roll-type heater, a plate-type heater, a fluidized bed dryer, an airborne dryer, a vacuum dryer, an infrared heating device, a far-infrared heating device, and a microwave heating device.

In the heat treatment according to the present embodiment, a method comprising adding Compound A to a thin sheet-like fiber raw material by impregnation or the like, and then heating the fiber raw material, or a method comprising heating a fiber raw material, while kneading or stirring the fiber raw material and Compound A using a kneader or the like, can be adopted. Thereby, the unevenness in the concentration of the Compound A in the fiber raw material can be suppressed, and phosphoric acid groups can be more uniformly introduced into the surface of cellulose fibers comprised in the fiber raw material. This is considered because, when water molecules move to the surface of the fiber raw material as drying advances, Compound A dissolved therein is attracted to the water molecules due to surface tension and as a result, Compound A also moves to the surface of the fiber raw material (specifically, the unevenness in the concentration of the Compound A occurs), and because such a phenomenon can be suppressed by adopting the aforementioned method.

As a heating device used for the heat treatment, for example, a device capable of always discharging moisture retained by slurry or moisture generated by the dehydration condensation (phosphoric acid esterification) reaction of Compound A with hydroxyl groups, etc. comprised in cellulose or the like in the fiber raw material, to the outside of the device system, is preferable. Such a heating device may be, for example, a ventilation-type oven. By always discharging moisture from the device system, in addition to being able to suppress a hydrolysis reaction of phosphoric acid ester

bonds, which is a reverse reaction of the phosphoric acid esterification, the acid hydrolysis of sugar chains in the fibers may also be suppressed. Thus, it becomes possible to obtain ultrafine cellulose fibers with a high axial ratio.

The time for the heat treatment is preferably 1 second or more and 300 minutes or less, more preferably 1 second or more and 1000 seconds or less, and further preferably 10 seconds or more and 800 seconds or less, for example, after moisture has been substantially removed from the fiber raw material. In the present embodiment, by setting the heating temperature and the heating time within an appropriate range, the amount of phosphoric acid groups introduced can be set within a preferred range.

The phosphoric acid group introduction step may be performed at least once, but may also be repeated two or more times. By performing the phosphoric acid group introduction step two or more times, many phosphoric acid groups can be introduced into the fiber raw material. In the present embodiment, as one example o

The amount of phosphoric acid groups introduced into the fiber raw material is, for example, per 1 g (mass) of the ultrafine cellulose fibers, preferably 0.10 mmol/g or more, more preferably 0.20 mmol/g or more, further preferably 0.50 mmol/g or more, and particularly preferably 1.00 mmol/g or more. On the other hand, the amount of phosphoric acid groups introduced into the fiber raw material is, for example, per 1 g (mass) of the ultrafine cellulose fibers, preferably 5.20 mmol/g or less, more preferably 3.65 mmol/g or less, and further preferably 3.00 mmol/g or less. By setting the amount of phosphoric acid groups introduced within the above-described range, it may become easy to perform fibrillation on the fiber raw material, and the stability of the ultrafine cellulose fibers can be enhanced.

< Carboxyl group introduction step >

The carboxyl group introduction step is carried out by performing ozonation, oxidation according to the Fenton method, or an oxidation treatment such as a TEMPO oxidation treatment, or by treating such a fiber raw material comprising cellulose with a compound having a carboxylic acid-derived group or a derivative thereof, or with an acid anhydride of the compound having a carboxylic acid-derived group or a derivative thereof.

Examples of the compound having a carboxylic acid-derived group may include, but are not particularly limited to, dicarboxylic acid compounds such as maleic acid, succinic acid, phthalic acid, fumaric acid, glutaric acid, adipic acid or itaconic acid, and tricarboxylic acid compounds such as citric acid or aconitic acid. In addition, examples of the derivative of the compound having a carboxylic acid-derived group may include, but are not particularly limited to, an imidized product of the acid anhydride of the compound having a carboxyl group and a derivative of the acid anhydride of the compound having a carboxyl group. Examples of the imidized product of the acid anhydride of the compound having a carboxyl group may include, but are not particularly limited to, imidized products of dicarboxylic acid compounds, such as maleimide, succinimide or phthalimide.

Examples of the acid anhydride of the compound having a carboxylic acid-derived group may include, but are not particularly limited to, acid anhydrides of dicarboxylic acid compounds, such as maleic anhydride, succinic anhydride, phthalic anhydride, glutaric anhydride, adipic anhydride, or itaconic anhydride. In addition, examples of the derivative of the acid anhydride of the compound having a carboxylic acid-derived group may include, but are not particularly limited to, acid anhydrides of the compounds having a carboxyl group, in which at least some hydrogen atoms are substituted with substituents such as alkyl groups or phenyl groups, such as dimethylmaleic anhydride, diethylmaleic anhydride, or diphenylmaleic anhydride.

In the case of performing a TEMPO oxidation treatment in the carboxyl group introduction step, the treatment is preferably carried out, for example, under conditions of pH 6 or more and pH 8 or less. Such a treatment is also referred to as a neutral TEMPO oxidation treatment. The neutral TEMPO oxidation treatment can be carried out, for example, by adding pulp used as a fiber raw material, nitroxy radical used as a catalyst, such as TEMPO (2,2,6,6-tetramethylpiperidin-1-oxyl), and sodium hypochlorite used as a sacrifice reagent to a sodium phosphate buffer (pH = 6.8). Moreover, by allowing sodium chlorite to coexist in the reaction system, aldehyde generated in the oxidation process can be efficiently oxidized to a carboxyl group.

Moreover, the TEMPO oxidation treatment may be carried out under conditions of pH 10 or more and pH 11 or less. Such a treatment is also referred to as an "alkaline TEMPO oxidation treatment." The alkaline TEMPO oxidation treatment can be carried out, for example, by adding nitroxy radicals such as TEMPO used as a catalyst, sodium bromide used as a co-catalyst, and sodium hypochlorite used as an oxidizer, to pulp as a fiber raw material.

The amount of carboxyl groups introduced into the fiber raw material is different depending on the types of the substituents. When carboxyl groups are introduced, for example, according to TEMPO oxidation, the amount of carboxyl groups introduced is, per 1 g (mass) of the ultrafine cellulose fibers, preferably 0.10 mmol/g or more, more preferably 0.20 mmol/g or more, further preferably 0.50 mmol/g or more, and particularly preferably 0.90 mmol/g or more. On the other hand, the amount of carboxyl groups introduced is, per 1 g (mass) of the ultrafine cellulose fibers, preferably 2.50 mmol/g or less, more preferably 2.20 mmol/g or less, and further preferably 2.00 mmol/g or less. Otherwise, when the substituents are carboxymethyl groups, the amount of carboxyl groups introduced may be, per 1 g (mass) of the ultrafine cellulose fibers, 5.8 mmol/g or less.

< Washing step >

In the method for producing ultrafine cellulose fibers according to the present embodiment, a washing step may be performed on the phosphoric acid group-introduced fibers, as necessary. The washing step is carried out by washing the phosphoric acid group-introduced fibers, for example, with water or an organic solvent. In addition, the washing step may be performed after each step as described below, and the number of washing operations performed in each washing step is not particularly limited.

< Alkali treatment step >

When the ultrafine cellulose fibers are produced, an alkali treatment may be performed on the fiber raw material between the phosphoric acid group introduction step and a defibration treatment step as described below. The method of the alkali treatment is not particularly limited. For example, a method of immersing the phosphoric acid group-introduced fibers in an alkaline solution may be applied.

The alkali compound contained in the alkaline solution is not particularly limited, and it may be an inorganic alkaline compound or an organic alkali compound. In the present embodiment, because of high versatility, for example, sodium hydroxide or potassium hydroxide is preferably used as an alkaline compound. In addition, the solvent contained in the alkaline solution may be either water or an organic solvent. Among others, the solvent contained in the alkaline solution is preferably water, or a polar solvent including a polar organic solvent such as alcohol, and is more preferably an aqueous solvent containing at least water. As an alkaline solution, for example, a sodium hydroxide aqueous solution or a potassium hydroxide aqueous solution is preferable, because of high versatility.

The temperature of the alkali solution in the alkali treatment step is not particularly limited, and for example, it is preferably 5°C or higher and 80°C or lower, and more preferably 10°C or higher and 60°C or lower. The time for immersion of the phosphoric acid group-introduced fibers in the alkali solution in the alkali treatment step is not particularly limited, and for example, it is preferably 5 minutes or more and 30 minutes or less, and more preferably 10 minutes or more and 20 minutes or less. The amount of the alkali solution used in the alkali treatment is not particularly limited, and for example, it is preferably 100% by mass or more and 100000% by mass or less, and more preferably 1000% by mass and 10000% by mass or less, with respect to the absolute dry mass of the phosphoric acid group-introduced fibers.

In order to reduce the amount of the alkaline solution used in the alkali treatment step, the phosphoric acid group-introduced fibers may be washed with water or an organic solvent after the phosphoric acid group introduction step and before the alkali treatment step. After the alkali treatment step and before the defibration step, the alkali-treated phosphoric acid group-introduced fibers are preferably washed with water or an organic solvent, from the viewpoint of the improvement of the handling ability.

< Acid treatment step >

When ultrafine cellulose fibers are produced, an acid treatment may be performed on the fiber raw material between the step of introducing phosphoric acid groups into the fiber raw material and the after-mentioned defibration treatment step. For example, a phosphoric acid group introduction step, an acid treatment, an alkali treatment, and a defibration treatment may be performed in this order.

Such an acid treatment method is not particularly limited, and for example, a method of immersing the fiber raw material in an acid solution containing an acid may be applied. The concentration of the used acid solution is not particularly limited, and for example, it is preferably 10% by mass or less, and more preferably 5% by mass or less. In addition, the pH of the used acid solution is not particularly limited, and for example, it is preferably a pH value of 0 or more and 4 or less, and more preferably a pH value of 1 or more and 3 or less. Examples of the acid contained in the acid solution that can be used herein may include inorganic acid, sulfonic acid, and carboxylic acid. Examples of the inorganic acid may include sulfuric acid, nitric acid, hydrochloric acid, hydrobromic acid, hydroiodic acid, hypochlorous acid, chlorous acid, chloric acid, perchloric acid, phosphoric acid, and boric acid. Examples of the sulfonic acid may include methanesulfonic acid, ethanesulfonic acid, benzenesulfonic acid, p-toluenesulfonic acid, and trifluoromethanesulfonic acid. Examples of the carboxylic acid may include formic acid, acetic acid, citric acid, gluconic acid, lactic acid, oxalic acid, and tartaric acid. Among these acids, it is particularly preferable to use hydrochloric acid or sulfuric acid.

The temperature of the acid solution used in the acid treatment is not particularly limited, and for example, it is preferably 5°C or higher and 100°C or lower, and more preferably 20°C or higher and 90°C or lower. The time for immersion of the fiber raw material in the acid solution in the acid treatment is not particularly limited, and for example, it is preferably 5 minutes or more and 120 minutes or less, and more preferably 10 minutes or more and 60 minutes or less. The amount of the acid solution used in the acid treatment is not particularly limited, and for example, it is preferably 100% by mass or more and 100000% by mass or less, and more preferably 1000% by mass or more and 10000% by mass or less, with respect to the absolute dry mass of the fiber raw material.

< Defibration treatment >

By performing a defibration treatment on the phosphoric acid group-introduced fibers in a defibration treatment step, ultrafine cellulose fibers are obtained. In the defibration treatment step, for example, a defibration treatment apparatus can be used. Such a defibration treatment apparatus is not particularly limited, and for example, a high-speed defibrator, a grinder (stone mill-type crusher), a high-pressure homogenizer, an ultrahigh-pressure homogenizer, a high-pressure collision-type crusher, a ball mill, a bead mill, a disc-type refiner, a conical refiner, a twin-screw kneader, an oscillation mill, a homomixer under high-speed rotation, an ultrasonic disperser, a beater or the like can be used. Among the above-described defibration treatment apparatuses, it is more preferable to use a high-speed defibrator, a high-pressure homogenizer, and an ultrahigh-pressure homogenizer, which are less affected by milling media, and are less likely to be contaminated.

In the defibration treatment step, for example, the phosphoric acid group-introduced fibers are preferably diluted with a dispersion medium to form a slurry. As a dispersion medium, water, and one type or two or more types selected from organic solvents such as polar organic solvents can be used. The polar organic solvent is not particularly limited, and for example, alcohols, polyhydric alcohols, ketones, ethers, esters, aprotic polar solvents, etc. are preferable. Examples of the alcohols may include methanol, ethanol, isopropanol, n-butanol, and isobutyl alcohol. Examples of the polyhydric alcohols may include ethylene glycol, propylene glycol, and glycerin. Examples of the ketones may include acetone and methyl ethyl ketone (MEK). Examples of the ethers may include diethyl ether, tetrahydrofuran, ethylene glycol monomethyl ether, ethylene glycol monoethyl ether, ethylene glycol mono n-butyl ether, and propylene glycol monomethyl ether. Examples of the esters may include ethyl acetate and butyl acetate. Examples of the aprotic polar solvents may include dimethyl sulfoxide (DMSO), dimethylformamide (DMF), dimethylacetamide (DMAc), and N-methyl-2-pyrrolidinone (NMP).

The solid concentration of the ultrafine cellulose fibers upon the defibration treatment can be determined, as appropriate. In addition, in a slurry obtained by dispersing the phosphoric acid group-introduced fibers in a dispersion medium, solids other than the phosphoric acid group-introduced fibers, such as hydrogen-binding urea, may be comprised.

As described above, a slurry containing ultrafine cellulose fibers can be obtained. The solid concentration in the slurry can be controlled, as appropriate, and for example, the solid concentration is preferably 0.1% by mass or more, and more preferably 0.5% by mass or more. On the other hand, the solid concentration is preferably 50% by mass or less, and more preferably 40% by mass or less.

(Resin component)

It is preferable that the sheet of the present invention further comprises a resin component. The resin component is preferably a water-soluble polymer. Examples of the water-soluble polymer may include: synthetic water-soluble polymers, such as a carboxy vinyl polymer, polyvinyl alcohol, an alkyl methacrylate-acrylic acid copolymer, polyvinyl pyrrolidone, sodium polyacrylate, polyethylene glycol, diethylene glycol, triethylene glycol, polyethylene oxide, propylene glycol, dipropylene glycol, polypropylene glycol, isoprene glycol, hexylene glycol, 1, 3-butylene glycol, polyacrylamide, and polyamine polyamide epihalohydrin; thickening polysaccharides, such as xanthan gum, guar gum, tamarind gum, carrageenan, locust bean gum, quince seed, alginic acid, pullulan, carrageenan, and pectin; cellulose derivatives, such as carboxymethyl cellulose, methyl cellulose, and hydroxyethyl cellulose; starches, such as cationized starch, raw starch, oxidized starch, etherified starch, esterified starch, and amylose; glycerins, such as glycerin, diglycerin, and polyglycerin; and hyaluronic acid and a metal salt of hyaluronic acid.

Among others, the water-soluble polymer is preferably polyvinyl alcohol, polyethylene oxide, or polyamine polyamide epihalohydrin, and is more preferably polyethylene oxide. Polyvinyl alcohol is also preferably modified polyvinyl alcohol, and the modified polyvinyl alcohol may be, for example, acetoacetyl group-modified polyvinyl alcohol. Examples of the polyamine polyamide epihalohydrin may include polyamine polyamide epichlorohydrin, polyamine polyamide epibromohydrin, and polyamine polyamide epiiodohydrin. Among these, polyamine polyamide epichlorohydrin is preferably used. The sheet of the present invention may comprise one type of, or two or more types of the above-described resin components.

The sheet of the present invention may comprise, as resin components, those other than the above-described water-soluble polymers. Examples of such a resin component may include a polypropylene resin, an acrylic resin, a polycarbonate resin, a polyester resin, a polyamide resin, a silicone resin, a fluorine resin, a chlorine resin, an epoxy resin, a melamine resin, a phenolic resin, a polyurethane resin, a diallyl phthalate resin, a polyol resin, a polyether resin, a cellulose derivative, and a polyethylene resin. Among these, a polypropylene resin is preferably used, and also, such a resin is preferably used in combination of the aforementioned water-soluble polymer. Moreover, the above-described resin component may be added in the form of an emulsion.

The content of the resin component in the sheet is preferably 1 part by mass or more, more preferably 5 parts

by mass or more, and further preferably 10 parts by mass or more, with respect to 100 parts by mass of ultrafine cellulose fibers in the sheet. On the other hand, the content of the resin in the sheet is preferably 100 parts by mass or less, more preferably 80 parts by mass or less, and further preferably 60 parts by mass or less, with respect to 100 parts by mass of the ultrafine cellulose fibers. By setting the content of the resin component in the sheet within the above-described range, the strength of the sheet can be enhanced. Moreover, by setting the content of the resin component in the sheet within the above-described range, the water content percentage of the sheet can be easily adjusted to a desired range. Specifically, since the amount of water absorbed can be suppressed by setting the content of the resin component in the sheet within the above-described range, when a sheet base paper is impregnated with a solution containing water, for example, in the after-mentioned step of producing a sheet, it becomes unnecessary to strictly control the time required for impregnating the base paper with the solution, and thus, handling ability can be enhanced upon the production of the sheet.

(External preparation for skin)

The sheet of the present invention preferably further comprises an external preparation for skin. Examples of such an external preparation for skin may include an oil base, a surfactant, alcohols, a moisturizer, a polymer/thickener/gelling agent, an antioxidant, an antiseptic, a fungicide, a chelating agent, a pH adjuster/acid/alkali, an ultraviolet absorber, a whitening agent, an exfoliating/dissolving agent, an antipruritic agent, an anti-inflammatory agent, an antiperspirant, a cooling agent, a reducing agent, an oxidizing agent, vitamins and the derivatives thereof, sugars and the derivatives thereof, organic acids, inorganic powders, a perfume, a dye, and a pigment. Among others, the external preparation for skin is preferably a beauty ingredient or a medicinal ingredient, and is more preferably a moisturizer, a whitening agent, an exfoliating/dissolving agent, an antipruritic agent, an anti-inflammatory agent, or vitamins and the derivatives thereof. The sheet of the present invention may comprise one type of, or two or more types of the above-described ingredients.

The content of the external preparation for skin in the sheet is preferably 0.0001% by mass or more, more preferably 0.001% by mass or more, and further preferably 0.01% by mass or more, with respect to the total mass of the sheet. On the other hand, the content of the external preparation for skin in the sheet is preferably 29% by mass or less, more preferably 25% by mass or less, and further preferably 20% by mass or less, with respect to the total mass of the sheet. By setting the content of the external preparation for skin within the above-described range, a wet sheet having excellent beauty effects or medicinal effects can be easily obtained. In addition, by setting the content of the external preparation for skin within the above-described range, a wet sheet having excellent handling ability and adhesiveness can be easily obtained.

(Other optional components)

The sheet of the present invention may comprise polyvalent metal salts. Examples of such polyvalent metal salts may include aluminum sulfate, magnesium sulfate, aluminum potassium sulfate, aluminum ammonium sulfate, and ferric chloride sulfate. Among others, the polyvalent metal salts are preferably at least one selected from aluminum sulfate and magnesium sulfate. By allowing the sheet of the present invention to comprise polyvalent metal salts, crosslinked structures are formed among ultrafine cellulose fibers in the sheet, so that the strength of the sheet can be enhanced. Thereby, the handling ability of the sheet can be further enhanced.

The sheet of the present invention may comprise a solvent other than water. The solvent may be an organic solvent. Examples of the organic solvent may include methanol, ethanol, n-propyl alcohol, isopropyl alcohol (IPA), 1-butanol, m-cresol, glycerin, acetic acid, pyridine, tetrahydrofuran (THF), acetone, methyl ethyl ketone (MEK), ethyl acetate, aniline, N-methyl-2-pyrrolidone (NMP), dimethyl sulfoxide (DMSO), N,N-dimethylformamide (DMF), hexane, cyclohexane, benzene, toluene, p-xylene, diethyl ether, chloroform, phenoxy ethanol, and butylene glycol. The content of the solvent other than water in the sheet is preferably 10% by mass or less, more preferably 5% by mass or less, and further preferably 1% by mass or less, with respect to the total mass of the sheet.

Examples of such other optional components may include organic ions, coupling agents, inorganic layered compounds, inorganic compounds, leveling agents, antifoaming agents, organic particles, lubricants, antistatic agents, magnetic powders, orientation promoters, plasticizers, dispersing agents, and crosslinkers. The sheet of the present invention may comprise one type of, or two or more types of the above-described ingredients.

The content of the above-described components in the sheet is preferably 10% by mass or less, more preferably 5% by mass or less, and further preferably 1% by mass or less, with respect to the total solid content mass in the sheet.

(Step of producing sheet)

The step of producing the sheet of the present invention comprises: a step of obtaining a slurry comprising

ultrafine cellulose fibers; a coating step of applying the slurry onto a base material or a papermaking step of making paper from the slurry; and a step of adding water to the sheet base paper obtained via the coating step or the papermaking step. Thereby, a wet sheet having a water content percentage of 70% by mass or more can be obtained. Among others, the step of producing the sheet preferably comprises: a step of obtaining a slurry comprising ultrafine cellulose fibers; a coating step of applying the slurry onto a base material; and a step of adding water to the sheet base paper obtained via the coating step.

The step of producing the sheet of the present invention preferably comprises a step of adding water to the sheet base paper obtained via the coating step or the papermaking step. In the coating step or the papermaking step, a step of drying the sheet base paper is preferably established. In the subsequent step of adding water, water is preferably added to a sheet that is in an absolute dry state or in a humidity controlled state, and more preferably, a sheet that is in an absolute dry state or in a humidity controlled state is impregnated with a solution containing water. Thereby, a wet sheet, in which the water content percentage is a predetermined value or more and water is uniformly retained, can be obtained. Besides, in the step of producing the sheet of the present invention, it is preferable that the sheet that is in an absolute dry state or in a dry state is processed into a desired shape, and that the sheet is then impregnated with a solution containing water. As such, in the step of producing the sheet of the present invention, since the sheet that is in an absolute dry state or in a dry state can be processed, for example, the sheet can be easily cut (punched) into a desired shape. Hence, the production efficiency of the sheet can be enhanced, and as a result, the cost of producing the sheet can be reduced. It is to be noted that the sheet that is in an absolute dry state is a sheet obtained by drying the sheet base paper obtained via the coating step or the papermaking step at 105°C for 24 hours, wherein the sheet has a water content percentage of 0% by mass. On the other hand, the sheet that is in a humidity controlled state is a sheet obtained by leaving at rest the sheet base paper obtained via the coating step or the papermaking step under conditions of 23°C and a relative humidity of 50% for 24 hours, wherein the sheet has a water content percentage of 15% by mass or less.

In the step of adding water to the sheet base paper obtained via the coating step or the papermaking step, a wet sheet is obtained. In this step, the amount of water to be added, impregnation time, and the like may be adjusted, so that the water content percentage of the wet sheet can be within a desired range. For instance, when the step of adding water is a step of impregnating the sheet base paper with a solution containing water, the time for immersing the sheet base paper in the solution can be set to be 1 second or longer and 60 minutes or shorter. Besides, as mentioned above, it is also possible to control the water content percentage by allowing the sheet to comprise a resin component. In this case, it is also possible to further extend the immersion time, etc.

The step of adding water may be a step of spraying water onto the sheet base paper obtained via the coating step or the papermaking step. In this case, the solution containing water is preferably sprayed onto the entire surface or a part of the sheet base paper. Moreover, the step of adding water may also be a step of coating the sheet base paper obtained via the coating step or the papermaking step with water.

It is to be noted that the solution containing water used in the step of adding water may comprise an external preparation for skin or other optional components, as necessary.

The step of producing the sheet of the present invention may comprise a step of impregnating the sheet base paper obtained via the coating step or the papermaking step with an aqueous solution containing polyvalent metal salts. The step of impregnating the sheet base paper with an aqueous solution containing polyvalent metal salts is preferably established before the step of adding water. After completion of the step of impregnating the sheet base paper with an aqueous solution containing polyvalent metal salts, the sheet base paper is converted to a sheet that is in an absolute dry state or in a dry state, and thereafter, the step of adding water is preferably established. By further establishing the step of impregnating the sheet base paper with an aqueous solution containing polyvalent metal in the step of producing the sheet, crosslinked structures are formed among ultrafine cellulose fibers comprised in the sheet, and the strength of the sheet can be enhanced. Thereby, the handling ability of the sheet can be further enhanced.

In the step of impregnating the sheet base paper with an aqueous solution containing polyvalent metal salts, it is preferable to adjust the concentration of polyvalent metal or the immersion time, depending on the amount of crosslinked structures to be formed. For example, when the concentration of polyvalent metal in an aqueous solution containing the polyvalent metal is set to be 1% to 10% by mass, the immersion time of the sheet base paper is preferably 10 seconds to 10 minutes. The aqueous solution containing polyvalent metal salts is preferably an aqueous solution containing salts derived from strong acid of di- or more-valent metal. Examples of the polyvalent metal salts may include aluminum sulfate, magnesium sulfate, aluminum potassium sulfate, aluminum ammonium sulfate, and ferric chloride sulfate. Among others, the polyvalent metal salts are preferably at least one selected from aluminum sulfate and magnesium sulfate.

After completion of the step of impregnating the sheet base paper with an aqueous solution containing polyvalent metal, a washing step is preferably established. By this washing step, unnecessary polyvalent metal can be removed. In addition, after completion of the washing step, a step of drying the sheet is preferably established. In the drying step, the sheet is preferably dried by being left at rest under a dry environment of 20°C to 100°C (for example, at a relative

humidity of 30% or less).

When the sheet comprises a resin component, such a resin component is preferably added in the step of obtaining a slurry comprising ultrafine cellulose fibers. The resin component is preferably added in the form of a resin solution, and such a resin solution is preferably an aqueous solution prepared by mixing a resin with water. Besides, after addition of the resin solution, the slurry may be heated, so that the dispersibility of the resin component may be enhanced.

< Coating step >

In the coating step, for example, a slurry comprising ultrafine cellulose fibers is applied onto a base material, and is then dried to form a sheet base paper, which is then detached from the base material, so as to obtain a sheet base paper. In addition, using a coating apparatus and a long base material, the sheet base papers can be continuously produced.

The material of the base material used in the coating step is not particularly limited. A base material having higher wettability to the slurry is preferable because shrinkage of the sheet base paper or the like upon drying is suppressed. It is preferable to select one from which a sheet base paper formed after drying can be easily detached. Of these, a resin film or plate, or a metal film or plate is preferable, but is not particularly limited thereto. Examples of the base material that can be used herein may include: resin films or plates, such as those made of acryl, polyethylene terephthalate, vinyl chloride, polystyrene, or polyvinylidene chloride; metal films or plates, such as those made of aluminum, zinc, copper, or iron; the aforementioned films or plates, the surfaces of which are subjected to an oxidation treatment; and stainless steel films or plates and brass films or plates.

When the slurry has a low viscosity and spreads on the base material in the coating step, a damming frame may be fixed and used on the base material in order to obtain a sheet base paper having a predetermined thickness and basis weight. The damming frame is not particularly limited, and for example, it is preferable to select ones from which the edges of the sheet base paper adhering thereto after drying can be easily detached. From such a viewpoint, frames molded from resin plates or metal plates are more preferable. In the present embodiment, examples of the frames that can be used herein may include: frames molded from resin plates, such as an acryl plate, a polyethylene terephthalate plate, a vinyl chloride plate, a polystyrene plate, or a polyvinylidene chloride plate; frames molded from metal plates, such as an aluminum plate, a zinc plate, a copper plate, or an iron plate; the aforementioned frames, the surfaces of which are subjected to an oxidation treatment; and frames molded from stainless steel plates, brass plates, etc.

A coater for applying the slurry onto the base material is not particularly limited, and examples of such a coater that can be used herein may include roll coaters, gravure coaters, die coaters, curtain coaters, and air doctor coaters. Among these, die coaters, curtain coaters, and spray coaters are particularly preferable because these coaters can provide more even thickness to the sheet base paper.

The slurry temperature and the ambient temperature applied upon application of the slurry onto the base material are not particularly limited, and for example, the temperatures are preferably 5°C or higher and 80°C or lower, more preferably 10°C or higher and 60°C or lower, further preferably 15°C or higher and 50°C or lower, and particularly preferably 20°C or higher and 40°C or lower. When the coating temperature is equal to or higher than the above-described lower limit value, it is possible to easily apply the slurry onto the base material. When the coating temperature is equal to or lower than the above-described upper limit value, it is possible to suppress volatilization of the dispersion medium during the coating.

In the coating step, it is preferable to apply the slurry onto the base material, so that the finished basis weight of the sheet base paper becomes preferably 10 g/$m^2$ or more and 200 g/$m^2$ or less, and more preferably 20 g/$m^2$ or more and 150 g/$m^2$ or less. By applying the slurry so that the basis weight can be within the above-described range, a sheet base paper having excellent strength can be obtained.

As described above, the coating step comprises a step of drying the slurry applied onto the base material. The step of drying the slurry is not particularly limited, and for example, a contactless drying method or a method of drying the sheet base paper while locking the sheet base paper, or a combination of these methods may be applied. The contactless drying method is not particularly limited, and for example, a method for drying by heating with hot air, infrared radiation, far-infrared radiation, or near-infrared radiation (a drying method by heating) or a method for drying in vacuum (a vacuum drying method) can be applied. Although the drying method by heating and the vacuum drying method may be combined with each other, the drying method by heating is usually applied. The drying with infrared radiation, far-infrared radiation, or near-infrared radiation is not particularly limited, and for example, it can be performed using an infrared apparatus, a far-infrared apparatus, or a near-infrared apparatus. The heating temperature applied in the drying method by heating is not particularly limited, and it is preferably 20°C or higher and 150°C or lower, and more preferably 25°C or higher and 105°C or lower. If the heating temperature is set to be equal to or higher than the above-described lower limit value, the dispersion medium can be rapidly volatilized. On the other hand, if the heating temperature is set to be equal to or lower than the above-described upper limit value, reduction in costs required for the heating and

suppression of the thermal discoloration of the cellulose fibers can be realized.

< Papermaking step >

The papermaking step is carried out by making a paper from a slurry using a paper machine. The paper machine used in the papermaking step is not particularly limited, and examples thereof may include continuous paper machines such as a Fourdrinier paper machine, a cylinder paper machine, and an inclined paper machine, and a multilayer combination paper machine, which is a combination thereof. A known papermaking method, such as papermaking by hand, may be adopted in the papermaking step.

The papermaking step is carried out by subjecting the slurry to wire-filtration and dehydration to obtain a sheet base paper that is in a wet state, and then pressing and drying this sheet base paper. The filter fabric used in the filtration and dehydration of the slurry is not particularly limited, and for example, a filter fabric, through which cellulose fibers do not pass and the filtration speed is not excessively slow, is more preferable. Such filter fabric is not particularly limited, and for example, a sheet, a woven fabric, or a porous membrane, each consisting of an organic polymer, is preferable. Preferred examples of the organic polymer may include, but are not particularly limited to, non-cellulose organic polymers such as polyethylene terephthalate, polyethylene, polypropylene, and polytetrafluoroethylene (PTFE). In the present embodiment, examples of the filter fabric may include a polytetrafluoroethylene porous membrane having a pore size of 0.1 $\mu$m or more and 20 $\mu$m or less, and a woven fabric made of polyethylene terephthalate or polyethylene having a pore size of 0.1 $\mu$m or more and 20 $\mu$m or less.

In the sheet formation step, the method for producing a sheet base paper from a slurry can be carried out, for example, using a production apparatus comprising a dewatering section for ejecting a slurry comprising ultrafine cellulose fibers onto the upper surface of an endless belt and then dewatering a dispersion medium contained in the ejected slurry to form a web, and a drying section for drying the web to produce a sheet base paper. The endless belt is provided across from the dewatering section to the drying section, and the web formed in the dewatering section is transferred to the drying section while being placed on the endless belt.

The dehydration method used in the papermaking step is not particularly limited, and for example, a dehydration method conventionally used for paper production may be applied. Among others, a method comprising performing dehydration using a Fourdrinier, cylinder, tilted wire, or the like and then performing dehydration using a roll press is preferable. In addition, the drying method used in the papermaking step is not particularly limited, and for example, a drying method used for paper production may be applied. Among others, a drying method using a cylinder dryer, a Yankee dryer, hot air drying, a near-infrared heater, or an infrared heater is more preferable.

(Intended use)

The intended use of the sheet of the present invention is not particularly limited, and the sheet of the present invention is preferably used, for example, as a cosmetic sheet. The cosmetic sheet may be, for example, a face mask. In particular, when the sheet of the present invention is used as a face mask, there is a case where the sheet is closely adhered onto the face, for example, by pulling the sheet upon the use thereof. Since the sheet of the present invention can exhibit sufficient strength and elongation in such usage, it is easily handled upon the use thereof without the breakage of the sheet.

Moreover, the sheet of the present invention can also be used as a sheet for adhering to a wound site, a medical sheet, a cooling sheet, a shock absorbing sheet, or a sheet for culturing bacteria, cells, tissues, etc.

Examples

The present invention will be more specifically described in the following examples. However, the following examples are not intended to limit the scope of the present invention.

[Example 1]

[Production of phosphorylated pulp]

The needle bleached kraft pulp manufactured by Oji Paper Co., Ltd. (solid content: 93% by mass; basis weight: 208 g/m$^2$, sheet-shaped; and Canadian Standard Freeness (CSF) measured according to JIS P 8121 after defibration is 700 ml) was used as a raw material pulp. A phosphorylation treatment was performed on this raw material pulp as follows. First, a mixed aqueous solution of ammonium dihydrogen phosphate and urea was added to 100 parts by mass (absolute dry mass) of the above raw material pulp, and the obtained mixture was adjusted to result in 45 parts by mass of the ammonium dihydrogen phosphate, 120 parts by mass of the urea and 150 parts by mass of water, so as to obtain

a chemical-impregnated pulp. Subsequently, the obtained chemical-impregnated pulp was heated in a hot-air dryer at 165°C for 200 seconds, so that phosphoric acid groups were introduced into cellulose in the pulp, thereby obtaining a phosphorylated pulp.

Subsequently, a washing treatment was performed on the obtained phosphorylated pulp. The washing treatment was carried out by repeating the operation to pour 10 L of ion exchange water onto 100 g (absolute dry mass) of the phosphorylated pulp to obtain a pulp dispersed solution, which was then uniformly dispersed by stirring, followed by filtration and dehydration. The washing was terminated at a time point at which the electric conductivity of the filtrate became 100 μS/cm or less.

Subsequently, a neutralization treatment was performed on the phosphorylated pulp after the washing as follows. First, the phosphorylated pulp after the washing was diluted with 10 L of ion exchange water, and then, while stirring, a 1 N sodium hydroxide aqueous solution was slowly added to the diluted solution to obtain a phosphorylated pulp slurry having a pH value of 12 or more and 13 or less. Thereafter, the phosphorylated pulp slurry was dehydrated, so as to obtain a neutralized phosphorylated pulp.

Subsequently, the above-described washing treatment was performed on the phosphorylated pulp after the neutralization treatment. The infrared absorption spectrum of the thus obtained phosphorylated pulp was measured by FT-IR. As a result, absorption based on the phosphoric acid groups was observed around 1230 $cm^{-1}$, and thus, addition of the phosphoric acid groups to the pulp was confirmed. Moreover, the obtained phosphorylated pulp was analyzed using an X-ray diffractometer. As a result, it was confirmed that there were typical peaks at two positions near $2\theta$ = 14° or more and 17° or less, and near $2\theta$ = 22° or more and 23° or less. Thus, the phosphorylated pulp was confirmed to have cellulose type I crystals.

[Defibration treatment]

Ion exchange water was added to the obtained phosphorylated pulp, so as to prepare a slurry having a solid concentration of 2% by mass. This slurry was treated using a wet atomization apparatus (manufactured by Sugino Machine Limited, Star Burst) at a pressure of 200 MPa twice to obtain an ultrafine cellulose fiber-dispersed solution A comprising ultrafine cellulose fibers. It was confirmed according to X-ray diffraction that these ultrafine cellulose fibers maintained cellulose type I crystals. Moreover, the fiber width of the ultrafine cellulose fibers was measured using a transmission electron microscope. As a result, the fiber width was 3 to 5 nm. Besides, the amount of phosphoric acid groups (the amount of strongly acidic groups) measured by the after-mentioned measurement method was 1.45 mmol/g.

< Measurement of amount of phosphoric acid groups >

The amount of phosphoric acid groups in the ultrafine cellulose fibers was measured by treating with an ion exchange resin, a cellulose fiber-containing slurry prepared by diluting an ultrafine cellulose fiber-dispersed solution comprising the ultrafine cellulose fibers as targets with ion exchange water to result in a content of 0.2% by mass, and then performing titration using alkali. In the treatment with the ion exchange resin, 1/10 by volume of a strongly acidic ion exchange resin (Amberjet 1024; manufactured by Organo Corporation; conditioned) was added to the aforementioned cellulose fiber-containing slurry, and the resultant mixture was shaken for 1 hour. Then, the mixture was poured onto a mesh having 90-μm apertures to separate the resin from the slurry. In the titration using alkali, a change in the electric conductivity value indicated by the slurry was measured while adding an aqueous solution of 0.1 N sodium hydroxide, once 30 seconds, in each amount of 50 μL, to the cellulose fiber-containing slurry after completion of the treatment with the ion exchange resin. Specifically, among the calculation results, the alkali amount (mmol) required in a region corresponding to the first region shown in Figure 1 was divided by the solid content (g) in the slurry to be titrated, so as to obtain the amount of phosphoric acid groups (mmol/g).

< Measurement of fiber width >

The fiber width of ultrafine cellulose fibers was measured by the following method. A supernatant of the ultrafine cellulose fiber-dispersed solution as obtained above by the treatment using a wet atomization apparatus was diluted with water, so that the concentration of the ultrafine cellulose fibers became 0.01% by mass or more and 0.1% by mass or less. The obtained solution was then added dropwise onto a hydrophilized carbon grid film. After drying, it was stained with uranyl acetate, and was then observed under a transmission electron microscope (manufactured by JEOL; JEOL-2000EX).

< Sheet formation >

Ion exchange water was added to the obtained ultrafine cellulose fiber-dispersed solution A to result in a solid

concentration of 0.5% by mass, so as to carry out concentration adjustment. Subsequently, an aqueous solution containing 0.5% by mass of polyethylene oxide (manufactured by SUMITOMO SEIKA CHEMICALS CO., LTD.; PEO-18) was added to this ultrafine cellulose fiber-dispersed solution A, so that the amount of the polyethylene oxide became 20 parts by mass with respect to 100 parts by mass of the ultrafine cellulose fibers, thereby obtaining a coating solution. Subsequently, the coating solution was weighed such that the basis weight of the obtained sheet (a layer constituted with the solid content of the above-described coating solution) that was in an absolute dry state became 50 g/m$^2$, and was then applied onto a commercially available acrylic plate, and thereafter, the acrylic plate was dried in a constant-temperature dryer at 50°C. In order to obtain the predetermined basis weight, a damming gold frame (a gold frame having an inside dimension of 180 mm × 180 mm, and a height of 5 cm) was arranged on the acrylic plate. Subsequently, the dried sheet was peeled from the above-described acrylic plate to obtain an ultrafine cellulose fiber-containing sheet 1.

After the dried ultrafine cellulose fiber-containing sheet 1 had been left at rest at 23°C, at a relative humidity of 50% for 24 hours, the water content percentage of the sheet was found to be 9.6% by mass. The water content percentage of the humidity-controlled sheet, after the sheet had been left at rest 23 °C at a relative humidity of 50% for 24 hours, was obtained by leaving the sheet at rest under conditions of 23°C, a relative humidity of 50% for 24 hours, then measuring the mass of the humidity-controlled sheet, then drying the sheet in a hot-air dryer at 105°C for 24 hours, then measuring the mass of the sheet in an absolute dry state, and then calculating the water content percentage according to the following equation:

Water content percentage [% by mass] after humidity control = (mass of humidity-controlled sheet – mass of sheet in an absolute dry stat) / mass of humidity-controlled sheet x 100.

< Moisturizing treatment >

The obtained ultrafine cellulose fiber-containing sheet 1 was immersed in ion exchange water at 23°C for 30 seconds to obtain an ultrafine cellulose fiber-containing wet sheet. It is to be noted that the water content percentage of the ultrafine cellulose fiber-containing wet sheet was obtained by immersing a 50-mm square ultrafine cellulose fiber-containing wet sheet in ion exchange water at 23°C for 30 seconds, then measuring the mass of the sheet after the immersion, then drying the sheet in a hot-air dryer at 105°C for 24 hours, then measuring the mass of the sheet in an absolute dry state, and then calculating the water content percentage according to the following equation. The measurement was repeatedly carried out 5 times, and a mean value thereof was defined to be the water content percentage of the ultrafine cellulose fiber-containing wet sheet.

Water content percentage [% by mass] of wet sheet = (mass of wet sheet after immersion in ion exchange water – absolute dry mass of sheet) / mass of wet sheet after immersion in ion exchange water x 100.

It is to be noted that the water content percentage of an ultrafine cellulose fiber-containing wet sheet with respect to a solid content mass was calculated according to the following equation:

Water content percentage (vs. solid content mass) [% by mass] of wet sheet = (mass of wet sheet after immersion in ion exchange water – absolute dry mass of sheet) / absolute dry mass of sheet x 100.

[Example 2]

An ultrafine cellulose fiber-containing wet sheet was obtained in the same manner as that of Example 1, with the exception that the immersion time applied in < Moisturizing treatment > was set to be 60 seconds.

[Example 3]

An ultrafine cellulose fiber-containing wet sheet was obtained in the same manner as that of Example 1, with

the exception that the immersion time applied in < Moisturizing treatment > was set to be 120 seconds.

[Example 4]

Acetoacetyl group-modified polyvinyl alcohol (manufactured by The Nippon Synthetic Chemical Industry Co., Ltd.; GOHSENX™ Z200; polymerization degree: 1200; saponification degree: 99 mol% or more) was added to ion exchange water to result in an amount of 10% by mass, and the obtained mixture was then stirred at 95°C for 1 hour for dissolution.

An ultrafine cellulose fiber-containing sheet 2 and an ultrafine cellulose fiber-containing wet sheet was obtained in the same manner as that of Example 2, with the exception that the ultrafine cellulose fiber-dispersed solution A was mixed with the acetoacetyl group-modified polyvinyl alcohol solution, so that the amount of the acetoacetyl group-modified polyvinyl alcohol became 40 parts by mass, with respect to 100 parts by mass of the ultrafine cellulose fibers.

[Example 5]

An aqueous solution containing 0.5% by mass of polyethylene oxide (manufactured by SUMITOMO SEIKA CHEMICALS CO., LTD.; PEO-18) was added in an amount of 20 parts by mass to 100 parts by mass of the ultrafine cellulose fiber-dispersed solution A having a sold concentration of 0.5% by mass. Thereafter, a polypropylene resin emulsion (manufactured by TOHO Chemical Industry Co., Ltd.; HYTEC P-5060P; particle diameter: 30 nm) was added to the dispersed solution, so that the amount of the polypropylene resin emulsion became 10 parts by mass, with respect to 100 parts by mass of the ultrafine cellulose fibers.

Thirty seconds after addition of the polypropylene resin emulsion, polyamine polyamide epichlorohydrin (manufactured by SEIKO PMC CORPORATION, wetting paper strength agent WS4030) was added to the reaction mixture, so that the amount of the polyamine polyamide epichlorohydrin became 0.4 parts by mass, with respect to 100 parts by mass of the ultrafine cellulose fibers. An ultrafine cellulose fiber-containing sheet 3 was obtained in the same manner as that of Example 1, with the exception that the sheet was formed from the aforementioned dispersed solution. The obtained dry ultrafine cellulose fiber-containing sheet 3 was immersed in ion exchange water at 23°C for 5 minutes to obtain an ultrafine cellulose fiber-containing wet sheet.

[Example 6]

An ultrafine cellulose fiber-containing sheet 4 and an ultrafine cellulose fiber-containing wet sheet was obtained in the same manner as that of Example 5, with the exception that the additive amount of the polyamine polyamide epichlorohydrin was set to be 0.2 parts by mass with respect to 100 parts by mass of the ultrafine cellulose fibers.

[Example 7]

A crosslinking treatment was carried out on an ultrafine cellulose fiber-containing sheet 1 in a dry state, which had been obtained in the same manner as that of Example 1, according to the following method. First, water was added to magnesium sulfate heptahydrate (manufactured by Kanto Chemical Co., Inc.) to prepare an aqueous solution having 5% by mass of magnesium sulfate (pure content). The ultrafine cellulose fiber-containing sheet 1 was immersed in this aqueous solution for 8 minutes, so as to carry out a crosslinking treatment using magnesium. Subsequently, the resulting sheet was immersed in ion exchange water for 15 minutes for washing. This washing operation was repeatedly carried out twice, and the sheet was then adhered to an acrylic plate, which was then dried in a chamber at 35°C and at a relative humidity of 15%, so as to obtain an ultrafine cellulose fiber-containing sheet 5 in a dry state.

The obtained ultrafine cellulose fiber-containing sheet 5 was immersed in ion exchange water at 23°C for 5 minutes to obtain an ultrafine cellulose fiber-containing wet sheet.

[Example 8]

An ultrafine cellulose fiber-containing sheet 6 and an ultrafine cellulose fiber-containing wet sheet was obtained in the same manner as that of Example 7, with the exception that the immersion time in the magnesium sulfate aqueous solution was set to be 3 minutes.

[Comparative Example 1]

An ultrafine cellulose fiber-containing wet sheet was obtained in the same manner as that of Example 5, with the exception that the immersion time applied in the moisturizing treatment was set to be 30 seconds.

[Comparative Example 2]

The sheet during the drying operation in the < Sheet formation > step of Example 1 was defined to be an ultrafine cellulose fiber-containing wet sheet. The water content percentage of the ultrafine cellulose fiber-containing wet sheet obtained in Comparative Example 2 was calculated according to the following equation:

Water content percentage [% by mass] = (mass of wet sheet during drying operation - absolute dry mass of sheet) / mass of wet sheet during drying operation x 100.

It is to be noted that the mass of the wet sheet during the drying operation was obtained by subtracting the mass of a sheet formation apparatus established in the constant-temperature dryer at 50°C in the < Sheet formation > step (a damming gold frame arranged on the acrylic plate) from the mass of the sheet formation apparatus and the entire mass of the sheet. In addition, the absolute dry mass of the sheet was defined to be the mass of the sheet obtained by drying the ultrafine cellulose fiber-containing wet sheet obtained during the drying operation at 105°C for 24 hours.

[Comparative Example 3]

The drying time was further reduced in the < Sheet formation > step of Comparative Example 2, and an ultrafine cellulose fiber-containing wet sheet, in which the entire water distribution was found to be uniform by visual observation, was obtained. It is to be noted that the water content percentage of this wet sheet was calculated according to the same method as that applied in Comparative Example 2.

[Comparative Example 4]

A pulp sheet consisting of the needle bleached kraft pulp manufactured by Oji Paper Co., Ltd. was defibrated using a swirl-type jet stream defibration apparatus, and thereafter, a fibrous sheet was formed using an air-laid web forming device. Thereafter, an aqueous binder solution of ethylene-vinyl acetate copolymer resin emulsion (manufactured by Sumika Chemtex Co., Ltd.; Sumikaflex 755; Tg - 15°C) was sprayed onto the fibrous sheet, so that the amount of the solid attached became 4.5 g/m$^2$. After that, hot air (ambient temperature: 150°C) was passed through the sheet, so that the fibers were allowed to mutually bind to one another. Furthermore, the fibrous sheet was inverted, and the aqueous binder solution was sprayed onto the surface opposite to the surface onto which the aqueous binder solution had been first sprayed, so that the amount of the solid attached became 44.5 g/m$^2$, and hot air (ambient temperature: 150°C) was passed through the sheet again, so as to obtain a dry non-woven fabric. The basis weight of the obtained non-woven fabric was 48 g/m$^2$.

[Evaluation]

[Evaluation of ultrafine cellulose fiber-containing sheet before moisturizing]

< Evaluation of punching workability >

An ultrafine cellulose fiber-containing sheet before being subjected to moisturizing was cut into a 100 mm square sheet, and was then left at rest at 23°C at a relative humidity of 50% for 24 hours. Thereafter, the sheet was subj ected to a punching test using a punching machine (manufactured by Fuji Shoko Machinery Co., Ltd., UD-5000). In this test, a pinnacle knife comprising a rectangular blade (60 mm long x 80 mm wide) for punching an outer frame, the corners of which were curved at R = 10 mm, and an elliptical blade (minor axis: 20 mm x major axis: 40 mm) for inner through hole was used as a punching knife. This punching knife was arranged, so that the short side of the blade for punching an outer frame could be vertical to the direction of travel of the sheet.

The punching test was carried out five times. Thereafter, the punched sheets and the pinnacle knife were observed, and were then evaluated according to the following criteria. It is to be noted that, when both sheet crack and dirt on the pinnacle knife were generated in a single test, it was counted as "once" as a set.

○: Neither chipping nor cracking was generated in all of the five tests, and no adhesives such as dirt were not found on the pinnacle knife.
Δ: Sheet crack and/or dirt on the pinnacle knife were generated in the sheet once in the five tests.
×: Sheet crack and/or dirt on the pinnacle knife were generated in the sheet twice or more in the five tests.

[Evaluation of ultrafine cellulose fiber-containing wet sheet]

< Density >

A 50 mm square ultrafine cellulose fiber-containing wet sheet after moisturizing treatment was dried in a hot-air dryer at 105°C for 24 hours, so that the sheet was absolutely dried. Thereafter, the thickness and basis weight of the sheet were measured, and the density (g/$cm^3$) of the sheet was then calculated.

< Tensile properties >

A moisturizing treatment was carried out in accordance with "7.2 Partial immersion method" of JIS P 8135, with the exceptions that an ultrafine cellulose fiber-containing sheet before being subjected to moisturizing was cut into a test piece with a size of 25 mm wide x 150 mm long, and that the immersion time was set to a time under individual moisturizing conditions. Thereafter, tensile strength (unit: N/m), tensile elastic modulus, and elongation were measured using a tension testing machine "Tensilon" (manufactured by A & D Company, Limited) in accordance with JIS P 8113, with the exception that a distance between holders was set to be 100 mm. It is to be noted that the immersion time of the sample used in the tensile property test was the same as those applied in individual examples and comparative examples. The tensile strength (unit: MPa) was calculated by dividing the tensile strength (unit: N/m) by the thickness of the test piece.

< Rubber hardness >

Rubber hardness was measured in accordance with JIS K 6253-3, with the exceptions that a moisturized ultrafine cellulose fiber-containing sheet was cut into a test piece with a size of 25 mm wide x 50 mm long and the test pieces were then laminated on one another to a thickness of 2 mm, and that the measurement time was set to be 30 seconds. For the measurement of rubber hardness, ASKER Rubber Hardness Tester Type E (manufactured by Kobunshi Keiki Co., Ltd.) was used.

< Haze >

The haze of an ultrafine cellulose fiber-containing wet sheet was measured in accordance with JIS K 7136, using a hazemeter (manufactured by MURAKAMI COLOR RESEARCH LABORATORY Co., Ltd.; HM-150).

< Ease of attachment of sheet >

An ultrafine cellulose fiber-containing wet sheet was cut into a 100 mm square, and this wet sheet was attached along the unevenness of the metacarpophalangeal joint of a human clenched fist. The ease of handling of the wet sheet upon the attachment was evaluated in accordance with the following criteria.

○: The wet sheet has moderate elongation and strength and is easily attached.
Δ: The wet sheet is broken if it is pulled strongly, or the wet sheet is extended too much and is somewhat hardly attached.
×: The wet sheet is hardly attached since it is easily broken, or it has strength but has poor elongation, and thus it is hardly attached.

< Visibility of air bubbles (evaluation of closely adhered state at initial stage) >

A 100 mm square wet sheet was attached along the unevenness of the metacarpophalangeal joint of a human clenched fist. Easy removal of air bubbles from the site between the skin and the sheet was evaluated in accordance with the following criteria.

○: Air bubbles can be confirmed from a distance of 30 cm or more by visual observation, and the air bubbles can be easily removed, so that the sheet can be closely adhered to the skin.
×: Air bubbles cannot be confirmed by visual observation, unless the sheet gets closer to a distance of within 10 cm. Otherwise, there are a large number of air bubbles caused by the unevenness of the sheet, and thus, it is impossible to remove all of the air bubbles and to closely adhere the sheet to the skin.

< Adhesiveness to skin (evaluation of adhesion persistence) >

A 100 mm square wet sheet was attached along the unevenness of the metacarpophalangeal joint of a human clenched fist, and thereafter, the opening and closing of the palm were carried out once. At the time, the degree of adhesiveness of the sheet was evaluated in accordance with the following criteria.

○: The sheet adheres to the skin and is not peeled.
Δ: The sheet is partially floated.
×: A half or more of the sheet is peeled.

[Table 1]

| | | Ex. 1 | Ex. 2 | Ex. 3 | Ex. 4 | Ex. 5 | Ex. 6 | Ex. 7 | Ex. 8 | Comp. Ex. 1 | Comp. Ex. 2 | Comp. Ex. 3 | Comp. Ex. 4 |
|---|---|---|---|---|---|---|---|---|---|---|---|---|---|
| Sheet | | CNF-containing sheet 1 | CNF-containing sheet 1 | CNF-containing sheet 1 | CNF-containing sheet 2 | CNF-containing sheet 3 | CNF-containing sheet 4 | CNF-containing sheet 5 | CNF-containing sheet 6 | CNF-containing sheet 3 | CNF-containing sheet 1 (during drying) | CNF-containing sheet 1 (during drying) | Dry non-woven fabric |
| Sheet composition (ratio when dried) | | CNF/PEO =100/20 | | | CNF/ modified PVA =100/40 | CNF/PEO/ PAE/PP = 100/20/ 0.4/10 | CNF/PEO/ PAE/PP =100/20/ 0.2/10 | CNF/PEO =100/20 Crosslinking after sheet formation | CNF/PEO =100/20 Crosslinking after sheet formation | CNF/PEO/ PAE/PP =100/20/ 0.4/10 | CNF/PEO =100/20 | | Pulp/ latex binder |
| Density (absolute dry state) | $g/cm^3$ | 1.11 | | | 1.35 | 1.02 | 1.09 | 1.19 | 1.21 | 1.02 | - | - | 0.04 |
| Basis weight (absolute dry state) | $g/m^2$ | 50 | | | 50 | 50 | 50 | 50 | 50 | 50 | - | - | 48 |
| Thickness (absolute dry state) | μm | 45.0 | | | 37.0 | 49.0 | 45.9 | 42.0 | 41.3 | 37.0 | - | - | 1200 |
| Water content percentage in humidity-controlled state (vs. sheet mass) | Mass % | 9.6 | | | 9.0 | 6.8 | 7.5 | 6.5 | 8.0 | 6.8 | | | 4.8 |
| Punchability (humidity controlled) | - | ○ | ○ | ○ | ○ | ○ | ○ | ○ | ○ | ○ | - | - | - |
| Water content percentage (vs. solid mass) | Mass % | 580 | 880 | 1250 | 680 | 440 | 800 | 240 | 610 | 180 | 256 (Mean value) | 1120 | 2020 |

(continued)

| | | Ex. 1 | Ex. 2 | Ex. 3 | Ex. 4 | Ex. 5 | Ex. 6 | Ex. 7 | Ex. 8 | Comp. Ex. 1 | Comp. Ex. 2 | Comp. Ex. 3 | Comp. Ex. 4 |
|---|---|---|---|---|---|---|---|---|---|---|---|---|---|
| Sheet | | CNF-containing sheet 1 | CNF-containing sheet 1 | CNF-containing sheet 1 | CNF-containing sheet 2 | CNF-containing sheet 3 | CNF-containing sheet 4 | CNF-containing sheet 5 | CNF-containing sheet 6 | CNF-containing sheet 3 | CNF-containing sheet 1 (during drying) | CNF-containing sheet 1 (during drying) | Dry nonwoven fabric |
| Sheet composition (ratio when dried) | | CNF/PEO =100/20 | | | CNF/ modified PVA =100/40 | CNF/PEO/ PAE/PP = 100/20/ 0.4/10 | CNF/PEO/ PAE/PP =100/20/ 0.2/10 | CNF/PEO =100/20 Crosslinking after sheet formation | CNF/PEO =100/20 Crosslinking after sheet formation | CNF/PEO/ PAE/PP =100/20/ 0.4/10 | CNF/PEO =100/20 | | Pulp/ latex binder |
| Water content percentage (vs. sheet mass) | Mass % | 85.3 | 89.8 | 92.6 | 87.2 | 81.5 | 88.9 | 70.6 | 85.9 | 64.3 | 71.9 | 91.8 | 95.3 |
| Tensile strength (humidity controlled) | MPa | 0.59 | 0.29 | 0.09 | 0.65 | 1.02 | 0.43 | 8.2 | 1.8 | 12.5 | - | - | 0.04 |
| Tensile elastic modulus (humidity controlled) | MPa | 1.45 | 1.37 | 0.7 | 0.87 | 6.1 | 1.3 | 38 | 22.4 | 52 | - | - | 0.31 |
| Elongation (humidity controlled) | % | 17.2 | 21.4 | 24.8 | 15.3 | 10.4 | 19.9 | 6.1 | 15.3 | 4.0 | - | - | 20.2 |
| Rubber hardness, Shore E 30 (humidity controlled) | /30 | 51 | 36 | 23 | 52 | 64 | 51 | 72 | 53 | 79 | - | - | - |
| Haze (humidity controlled) | % | 0.9 | 1.0 | 1.1 | 5.9 | 6.7 | 7.1 | 6.5 | 7.3 | 6.8 | - | - | - |

(continued)

| | | Ex. 1 | Ex. 2 | Ex. 3 | Ex. 4 | Ex. 5 | Ex. 6 | Ex. 7 | Ex. 8 | Comp. Ex. 1 | Comp. Ex. 2 | Comp. Ex. 3 | Comp. Ex. 4 |
|---|---|---|---|---|---|---|---|---|---|---|---|---|---|
| Sheet | | CNF-containing sheet 1 | CNF-containing sheet 1 | CNF-containing sheet 1 | CNF-containing sheet 2 | CNF-containing sheet 3 | CNF-containing sheet 4 | CNF-containing sheet 5 | CNF-containing sheet 6 | CNF-containing sheet 3 | CNF-containing sheet 1 (during drying) | CNF-containing sheet 1 (during drying) | Dry nonwoven fabric |
| Sheet composition (ratio when dried) | | CNF/PEO =100/20 | | | CNF/ modified PVA =100/40 | CNF/PEO/ PAE/PP = 100/20/ 0.4/10 | CNF/PEO/ PAE/PP =100/20/ 0.2/10 | CNF/PEO =100/20 Crosslinking after sheet formation | CNF/PEO =100/20 Crosslinking after sheet formation | CNF/PEO/ PAE/PP =100/20/ 0.4/10 | CNF/PEO =100/20 | | Pulp/ latex binder |
| Ease of attachment (humidity controlled) | - | ○ | ○ | Δ | ○ | ○ | ○ | Δ | ○ | × | - | - | - |
| Visibility of airbubbles (initially adhered state) (humidity controlled) | - | ○ | ○ | ○ | ○ | ○ | ○ | ○ | ○ | × | | | × |
| Adhesiveness to skin (adhesion persistence) (humidity controlled) | - | ○ | ○ | ○ | ○ | ○ | ○ | Δ | ○ | × | - | - | Δ |

The ultrafine cellulose fiber-containing wet sheets obtained in the Examples could be easily adhered to the skin, and were excellent in terms of adhesiveness to the skin (initial adhesiveness and adhesion persistence). In addition, the ultrafine cellulose fiber-containing wet sheets obtained in the Examples were also excellent in terms of transparency.

Moreover, the ultrafine cellulose fiber-containing wet sheets obtained in the Examples were excellent in terms of workability in a dry state, and in particular, were excellent in terms of punching workability. These effects cannot be obtained, for example, from bio cellulose-based ultrafine cellulose fiber-containing wet sheets.

On the other hand, the ultrafine cellulose fiber-containing wet sheet obtained in Comparative Example 1 had a low water content percentage, was not easily attached, and also had poor adhesiveness. The ultrafine cellulose fiber-containing wet sheet obtained in Comparative Example 2 comprised dry parts and watery parts, and apparently, water distribution was not uniform even by visual observation, and a sample that was suitable for the evaluations could not be obtained. Moreover, the sheet of Comparative Example 3 was extremely fragile, and could not be peeled from the acrylic plate. As such, a sample that was suitable for the evaluations could not be obtained. When the sheet obtained in Comparative Example 4 was attached to the surface of the skin, a large number of air bubbles were generated, and the air bubbles could not be removed, and thus, the sheet could not be closely adhered to the skin.

## Claims

**1.** A sheet comprising cellulose fibers having a fiber width of 1000 nm or less and water, wherein
the sheet is gelatinous,
the cellulose fibers have ionic substituents,
the water content percentage is 70% by mass or more with respect to the total mass of the sheet, and
the tensile strength is 0.08 MPa or more.

**2.** The sheet according to claim 1, wherein the fiber width of the cellulose fibers is 8 nm or less.

**3.** The sheet according to claim 1 or 2, which has a tensile elastic modulus of 0.5 MPa or more.

**4.** The sheet according to any one of claims 1 to 3, which has an elongation of 5.0% or more.

**5.** The sheet according to any one of claims 1 to 4, which has a haze of 20.0% or less.

**6.** The sheet according to any one of claims 1 to 5, wherein the density of the sheet in an absolute dry state is 0.5 $g/cm^3$ or more.

**7.** The sheet according to any one of claims 1 to 6, wherein the ionic substituents are phosphoric acid groups or phosphoric acid group-derived substituents.

**8.** The sheet according to any one of claims 1 to 7, which further comprises a resin component.

**9.** The sheet according to any one of claims 1 to 8, which further comprises an external preparation for skin.

**10.** The sheet according to any one of claims 1 to 9, which is used as a cosmetic sheet.

[Figure 1]

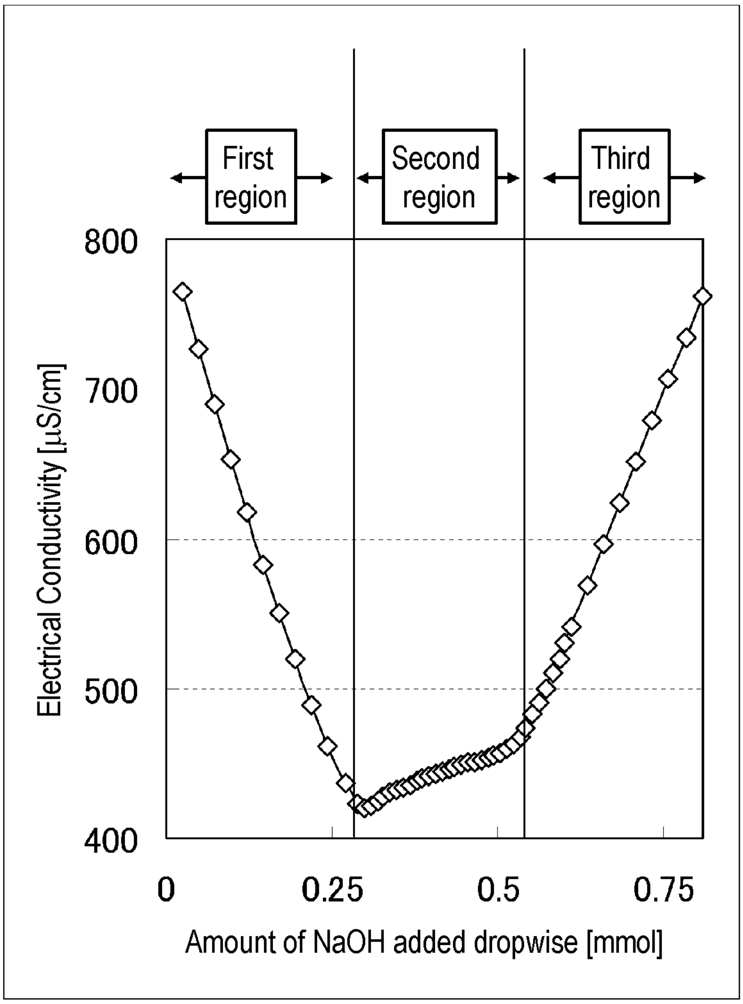
First region
Second region
Third region
Electrical Conductivity [µS/cm]
800
700
600
500
400
0
0.25
0.5
0.75
Amount of NaOH added dropwise [mmol]

[Figure 2]

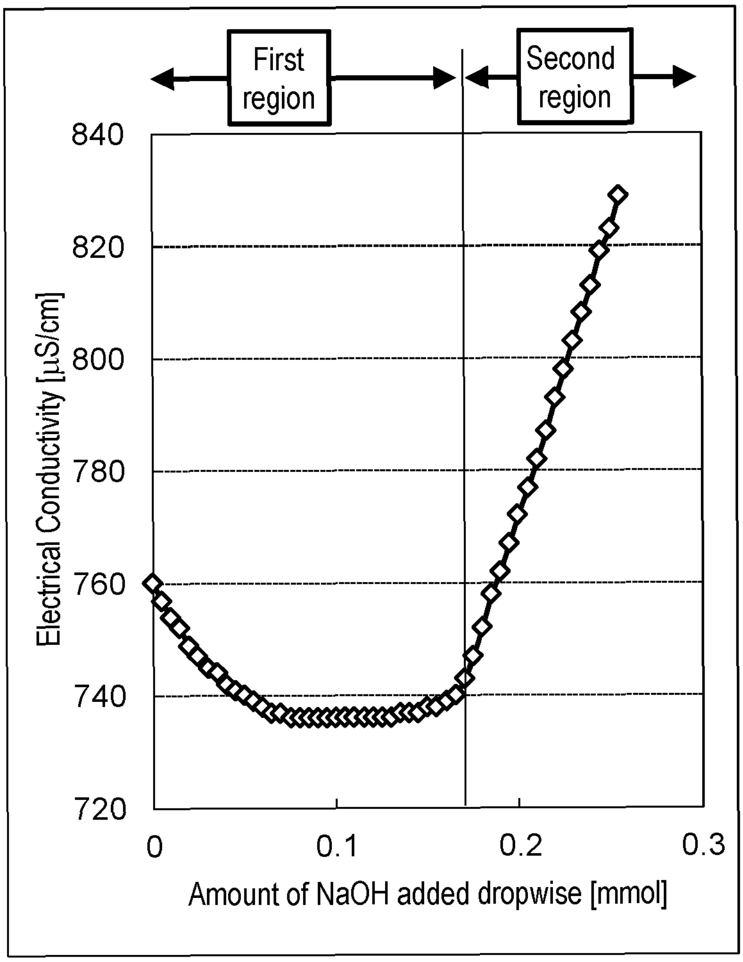
First region
Second region
840
820
800
780
760
740
720
Electrical Conductivity [µS/cm]
0
0.1
0.2
0.3
Amount of NaOH added dropwise [mmol]

## INTERNATIONAL SEARCH REPORT

International application No.
PCT/JP2019/000214

A. CLASSIFICATION OF SUBJECT MATTER
Int.Cl. D21H27/00(2006.01)i, A61K8/02(2006.01)i, A61K8/73(2006.01)i, A61Q19/00(2006.01)i, D21H11/18(2006.01)i, C08J9/40(2006.01)n

According to International Patent Classification (IPC) or to both national classification and IPC

B. FIELDS SEARCHED
Minimum documentation searched (classification system followed by classification symbols)
Int.Cl. D21H11/00-27/42, C08J3/075, C08J9/40, A61K8/00-8/99, A61Q19/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
Japio-GPG/FX, JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X<br>A | JP 6211160 B1 (DAIO PAPER CORPORATION) 11 October 2017, claims, paragraphs [0022]-[0025], examples, particularly, paragraphs [0077], [0078]<br>& WO 2018/061306 A1 | 1-8<br>9, 10 |
| A | US 2016/0186377 A1 (INNOVATECH ENGINEERING, LLC) 30 June 2016, claims, paragraph [0041]<br>& US 2018/0044856 A1 | 1-10 |
| A | JP 2011-127267 A (ASAHI KASEI FIBERS CORPORATION) 30 June 2011, claims, paragraphs [0001], [0017]-[0019], examples<br>(Family: none) | 1-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29.03.2019 | 09.04.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/000214

C (Continuation). DOCUMENTS CONSIDERED TO BE RELEVANT

| Category* | Citation of document, with indication, where appropriate, of the relevant passages | Relevant to claim No. |
|---|---|---|
| A | JP 2016-210893 A (JAPAN ADVANCED INSTITUTE OF SCIENCE & TECHNOLOGY HOKURIKU) 15 December 2016, claim 3<br>(Family: none) | 1-10 |
| A | JP 2006-298846 A (TOYO SHINYAKU CO., LTD.) 02 November 2006, claims, paragraph [0018], examples<br>(Family: none) | 1-10 |
| A | KR 10-2017-0120019 A (LG HOUSEHOLD & HEALTH CARE LTD.) 30 October 2017, claims, paragraphs [0003], [0035]<br>(Family: none) | 1-10 |
| P, A | JP 2018-002668 A (DAI ICHI KOGYO SEIYAKU CO., LTD.) 11 January 2018, claims, paragraphs [0019], [0056], examples<br>(Family: none) | 1-10 |
| P, A | WO 2018/109275 A1 (UPM-KYMMENE CORPORATION) 21 June 2018, claims, page 13, line 31 to page 14, line 28, page 27, line 27 to page 28, line 13, examples<br>& EP 3335740 A1 | 1-10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2017150110 A
- JP 2014111639 A
- JP 2005124916 A
- JP 2014205924 A

### Non-patent literature cited in the description

- **SEAGAL et al.** *Textile Research Journal,* 1959, vol. 29, 786